# EUROPEAN PATENT SPECIFICATION

(11) **EP 1 280 806 B1**
(45) Date of publication and mention of the grant of the patent: **04.08.2004**
(21) Application number: 01926294.8
(22) Date of filing: 26.04.2001
(51) Int. Cl.: C07D 495/04, A61K 31/513, A61P 11/00, A61P 37/06

(54) **THIENO 2,3-d]PYRIMIDINEDIONES AND THEIR USE AS PHARMACEUTICALS**
THIENO[2,3-d]PYRIMIDINDIONE UND IHRE VERWENDUNG ALS PHARMAZEUTISCHE MITTEL
THIENO 2,3-D]PYRIMIDINEDIONES ET LEUR UTILISATION EN TANT QUE PRODUITS PHARMACEUTIQUES

(30) Priority: 04.05.2000 GB 0010657; 21.07.2000 GB 0017795
(43) Date of publication of application: 05.02.2003
(73) Proprietor: AstraZeneca AB, 151 85 Södertälje (SE)
(72) Inventor: BANTICK, John, 78 Melton Road, Loughborough LE12 5AG (GB); INGALL, Anthony, AstraZeneca R & D Charnwood, Loughborough LE11 5RH (GB); PERRY, Matthew, AstraZeneca R & D Charnwood, Loughborough LE11 5RH (GB); REYNOLDS, Rachel, AstraZeneca R & D Charnwood, Loughborough LE11 5RH (GB)
(74) Representative: Tinsley, Rachel Maria
(86) International application number: PCT/SE2001/000907
(87) International publication number: WO 2001/083489

(56) References cited:
- EP-A1- 0 640 606
- WO-A1-00/12514
- WO-A1-97/07119
- WO-A1-98/54190
- DD-A5- 293 824
- PATENT ABSTRACTS OF JAPAN & JP 01 213 284 A (SANKYO CO LTD) 28 August 1989

## Description

The present invention relates to thieno[2,3-d]pyrimidinediones, processes for their preparation, pharmaceutical compositions containing them and their use in therapy.

Certain thieno[2,3-d]pyrimidinediones and their use in therapy is described in WO 00/12514 and WO 01/83489.

In accordance with the present invention, there is provided a compound of general formula 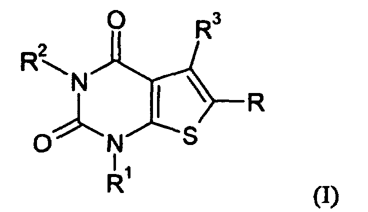 wherein:
R is -C(O)Ar¹, -C(R⁴)(R⁵)Ar¹ or Ar³;
Ar¹ represents a 5- to 10-membered aromatic ring system wherein up to 3 ring atoms may be heteroatoms independently selected from nitrogen, oxygen and sulphur, the ring system being optionally substituted by one or more substituents independently selected from C₁₋₄ alkyl, C₁₋₄ alkoxy, halogen, trifluoromethyl, oxo, nitro, cyano, NR⁶R⁷ and - CH₂NR⁸R⁹;
R¹ and R² each independently represent a hydrogen atom, C₁₋₆ alkyl, C₃₋₆ alkenyl, CH₂C₃₋₅ cycloalkyl or C₃₋₆ cycloalkyl;
R³ represents a group X-Ar²;
X represents a group S(O)ₙ, C(O) or CH(OH);
n is 0, 1 or 2;
Ar² represents a pyrrolyl, thiazolyl, thiadiazolyl, oxazolyl, imidazolyl or triazolyl ring, the ring being optionally substituted by one or more substituents independently selected from C₁₋₄ alkyl, C₁₋₄ alkoxy, C₁₋₄ alkylthio, halogen, trifluoromethyl, oxo, hydroxyl, amino (NH₂), nitro, cyano and benzyl;
R⁴ represents a hydrogen atom or C₁₋₄ alkyl (e.g. methyl, ethyl, n-propyl or n-butyl);
R⁵ represents a hydrogen atom or hydroxyl group;
R⁶ and R⁷ each independently represent a hydrogen atom or C₁₋₄ alkyl, or together with the nitrogen atom to which they are attached form a 5- to 7-membered saturated heterocyclic ring;
R⁸ and R⁹ each independently represent a hydrogen atom or C₁₋₄ alkyl, or together with the nitrogen atom to which they are attached form a 5- to 7-membered saturated heterocyclic ring; and
Ar³ represents acenaphthenyl, indanyl or fluorenyl, each of which may be optionally substituted by one or more substituents independently selected from C₁₋₄ alkyl, C₁₋₄ alkoxy, halogen or trifluoromethyl;
or a pharmaceutically acceptable salt or solvate thereof.

In the present specification, unless otherwise indicated, an alkyl or alkenyl group or an alkyl or alkenyl moiety in a substituent group may be linear or branched. It will be appreciated that when R represents a group -C(R⁴)(R⁵)Ar¹, R⁵ may represent a hydroxyl group only when Ar¹ is bonded to -C(R⁴)(R⁵) through a carbon atom and not a heteroatom. Furthermore, it should be understood that when R represents a group -C(O)Ar¹, Ar¹ is bonded through a carbon atom and not a heteroatom to the moiety -C(O).

R preferably represents -C(R⁴)(R⁵)Ar¹. R⁴ and R⁵ preferably both represent a hydrogen atom.

Ar¹ represents a 5- to 10-membered aromatic ring system wherein up to 3 ring atoms may be heteroatoms independently selected from nitrogen, oxygen and sulphur, the ring system being optionally substituted by one or more (e.g. one, two, three or four) substituents independently selected from C₁₋₄ alkyl (e.g. methyl, ethyl, n-propyl or n-butyl), C₁₋₄ alkoxy (e.g. methoxy, ethoxy, n-propoxy or n-butoxy), halogen (e.g. fluorine, chlorine, bromine or iodine), trifluoromethyl, oxo, nitro, cyano, NR⁶R⁷ and -CH₂NR⁸R⁹. Preferred substituents to use are C₁₋₄ alkyl, halogen and trifluoromethyl.

The aromatic ring system may be monocyclic or polycyclic (e.g. bicyclic), examples of which include phenyl, naphthyl, pyrazolyl, thienyl, oxazolyl, imidazolyl, pyridinyl, pyridopyrrolyl, benzimidazolyl, indazolyl, benzothiazolyl, benzoxazolyl, thiazolyl and benzotriazolyl. Preferably, the aromatic ring system is monocyclic and 5- or 6-membered, especially phenyl.

R¹ and R² each independently represent a hydrogen atom, C₁₋₆, preferably C₁₋₄, alkyl (e.g. methyl, ethyl, n-propyl, 1-methylethyl, n-butyl, 2-methylpropyl, n-pentyl or n-hexyl), C₃₋₆, preferably C₃₋₄, alkenyl (e.g. 1-propenyl, 1-butenyl, 1-pentenyl or 1-hexenyl), CH₂C₃₋₅ cycloalkyl (cyclopropylmethyl, cyclobutylmethyl or cyclopentylmethyl) or C₃₋₆, preferably C₅₋₆, cycloalkyl (cyclopropyl, cyclobutyl, cyclopentyl or cyclohexyl).

Most preferably R¹ and R² each independently represent a C₁₋₄ alkyl group.

R⁶ and R⁷ each independently represent a hydrogen atom or C₁₋₄ alkyl (e.g. methyl, ethyl, n-propyl or n-butyl), or together with the nitrogen atom to which they are attached form a 5- to 7-membered saturated heterocyclic ring.

R⁸ and R⁹ each independently represent a hydrogen atom or C₁₋₄ alkyl (e.g. methyl, ethyl, n-propyl or n-butyl), or together with the nitrogen atom to which they are attached form a 5- to 7-membered saturated heterocyclic ring.

Ar² represents a pyrrolyl, thiazolyl, thiadiazolyl, oxazolyl, imidazolyl or triazolyl ring, the ring being optionally substituted by one or more (e.g. one, two or three) substituents independently selected from C₁₋₄ alkyl (e.g. methyl, ethyl, n-propyl or n-butyl), C₁₋₄ alkoxy (e.g. methoxy, ethoxy, n-propoxy or n-butoxy), C₁₋₄ alkylthio (e.g. methylthio, ethylthio, n-propylthio or n-butylthio), halogen (e.g. fluorine, chlorine, bromine or iodine), trifluoromethyl, oxo, hydroxyl, amino, nitro, cyano and benzyl. A preferred substituent is C₁₋₄ alkyl.

Ar³ represents acenaphthenyl, indanyl or fluorenyl, each of which may be optionally substituted by one or more (e.g. one, two or three) substituents independently selected from C₁₋₄ alkyl (e.g. methyl, ethyl, n-propyl or n-butyl), C₁₋₄ alkoxy (e.g. methoxy, ethoxy, n-propoxy or n-butoxy), halogen (e.g. fluorine, chlorine, bromine or iodine) and trifluoromethyl.

Preferred compounds of the invention include:
1-Isobutyl-3-methyl-5-[(1-methyl-1H-pyrrol-2-yl)carbonyl]-6-[2-(trifluoromethyl)benzyl]-thieno[2,3-d]pyrimidine-2,4(1H,3H)-dione,
3-Methyl-1-(2-methylpropyl)-5-(1H-1,2,4-triazol-3-ylthio)-6-[2-(trifluoromethyl)benzoyl]-thieno[2,3-d]pyrimidine-2,4(1 H,3H)-dione,
3-Methyl-1-(2-methylpropyl)-5-(1H-1,2,4-triazol-3-ylthio)-6-[[2-(trifluoromethyl)phenyl]methyl]-thieno[2,3-d]pyrimidine-2,4(1H,3H)-dione,
3-Methyl-1-(2-methylpropyl)-5-(1H-1,2,4-triazol-3-ylsulfonyl)-6-[[2-(trifluoromethyl)phenyl]methyl]-thieno[2,3-d]pyrimidine-2,4(1 H,3H)-dione,
3-Methyl-1-(2-methylpropyl)-5-(2-thiazolylthio)-6-[2-(trifluoromethyl)henzoyl]-thieno[2,3-d]pyrimidine-2,4(1H,3H)-dione,
3-Methyl-1-(2-methylpropyl)-5-[[5-(methylthio)-1,3,4-thiadiazol-2-yl]thio]-6-[2-(trifluoromethyl)benzoyl]-thieno[2,3-d]pyrimidine-2,4(1H,3H)-dione,
3-Methyl-5-[(4-methyl-2-oxazolyl)thio]-1-(2-methylpropyl)-6-[2-(trifluoromethyl)benzoyl]-thieno[2,3-d]pyrimidine-2,4(1H,3H)-dione,
3-Methyl-1-(2-methylpropyl)-5-[(5-methyl-1H-1,2,4-triazol-3-yl)thio]-6-[2-(trifluoromethyl)benzoyl]-thieno[2,3-d]pyrimidine-2,4(1 H,3H)-dione,
3-Methyl-1-(2-methylpropyl)-5-[(5-hydroxy-4-(1-methylethyl)-1H-1,2,4-triazol-3-yl)thio]-6-[2-(trifluoromethyl)benzoyl]-thieno[2,3-d]pyrimidine-2,4(1H,3H)-dione,
3-Methyl-1-(2-methylpropyl)-5-[(5-methyl-1,3,4-thiadiazol-2-yl)thio]-6-[2-(trifluoromethyl)benzoyl]-thieno[2,3-d]pyrimidine-2,4(1H,3H)-dione,
5-(1H-Imidazol-2-ylthio)-3-methyl-1-(2-methylpropyl)-6-[2-(trifluoromethyl)benzoyl]-thieno[2,3-d]pyrimidine-2,4(1H,3H)-dione,
5-[[4-(1,1-Dimethylethyl)-4H-1,2,4-triazol-3-yl]thio]-3-methyl-1-(2-methylpropyl)-6-[2-(trifluoromethyl)benzoyl]-thieno[2,3-d]pyrimidine-2,4(1H,3H)-dione,
5-[(5-Amino-1,3,4-thiadiazol-2-yl)thio]-3-methyl-1-(2-methylpropyl)-6-[2-(trifluoromethyl)benzoyl]-thieno[2,3-d]pyrimidine-2,4(1H,3H)-dione,
3-Methyl-1-(2-methylpropyl)-5-(1,3,4-thiadiazol-2-ylthio)-6-[2-(trifluoromethyl)benzoyl]-thieno[2,3-d]pyrimidine-2,4(1H,3H)-dione,
6-[Hydroxy[2-(trifluoromethyl)phenyl)methyl]-5-(1H-imidazol-2-ylthio)-3-methyl-1-(2-methylpropyl)-thieno[2,3-d]pyrimidine-2,4(1H,3H)-dione,
6-[Hydroxy[2-(trifluoromethyl)phenyl]methyl]-3-methyl-1-(2-methylpropyl)-5-(1H-1,2,4-triazol-3-ylthio)-thieno[2,3-d]pyrimidine-2,4(1H,3H)-dione,
3-Methyl-1-(2-methylpropyl)-5-(2-thiazolylthio)-6-[[2-(trifluoromethyl)-phenyl]methyl]-thieno[2,3-d]pyrimidine-2,4(1H,3H)-dione,
3-Methyl-1-(2-methylpropyl)-5-[(5-methyl-1H-1,2,4-triazol-3-yl)thio]-6-[[2-(trifluoromethyl)phenyl]methyl]-thieno[2,3-d]pyrimidine-2,4(1H,3H)-dione dihydrochloride,
5-(1H-Imidazol-2-ylthio)-3-methyl-1-(2-methylpropyl)-6-[[2-(trifluoromethyl)phenyl]methyl]-thieno[2,3-d]pyrimidine-2,4(1H,3H)-dione,
3-Methyl-1-(2-methylpropyl)-5-(1,3,4-thiadiazol-2-ylthio)-6-[2-(trifluoromethyl)benzyl]-thieno[2,3-d]pyrimidine-2,4(1H,3H)-dione,
5-[(5-Amino-1,3,4-thiadiazol-2-yl)thio]-3-methyl-1-(2-methylpropyl)-6-[2-(trifluoromethyl)benzyl]-thieno[2,3-d]pyrimidine-2,4(1H,3H)-dione,
3-Methyl-1-(2-methylpropyl)-5-[(5-methyl-1H-1,2,4-triazol-3-yl)sulfonyl]-6-[[2-(trifluoromethyl)phenyl]methyl]-thieno[2,3-d]pyrimidine-2,4(1H,3H)-dione,
5-(1H-Imidazol-2-ylsulfonyl)-3-methyl-1-(2-methylpropyl)-6-[[2-(trifluoromethyl)phenyl]methyl]-thieno[2,3-d]pyrimidine-2,4(1H,3H)-dione,
6-[Hydroxy[2-(trifluoromethyl)phenyl]methyl]-3-methyl-1-(2-methylpropyl)-5-(1H-1,2,4-triazol-3-ylsulfonyl)-thieno[2,3-d]pyrimidine-2,4(1H,3H)-dione,
3-Methyl-1-(2-methylpropyl)-5-(1,3-thiazol-2-ylsulfonyl)-6-[2-(trifluoromethyl)benzyl]-thieno[2,3-d]pyrimidine-2,4(1H,3H)-dione,
3-Methyl-1-(2-methylpropyl)-5-(1,3,4-thiadiazol-2-ylsulfonyl)-6-[2-(trifluoromethyl)benzyl]-thieno[2,3-d]pyrimidine-2,4(1H,3H)-dione,
5-[(3-Bromo-1H-1,2,4-triazol-5-yl)thio]-3-methyl-1-(2-methylpropyl)-6-[2-(trifluoromethyl)phenylmethyl]-thieno[2,3-d]pyrimidine-2,4(1H,3H)-dione,
3-Methyl-1-(2-methylpropyl)-5-[(5-oxo-4,5-dihydro-1H-1,2,4-triazol-3-yl)thio]-6-[2-(trifluoromethyl)phenylmethyl]-thieno[2,3-d]pyrimidine-2,4(1H,3H)-dione,
3-Methyl-1-(1-methylethyl)-5-(1,3-thiazol-2-ylthio)-6-[2-(trifluoromethyl)benzyl]-thieno[2,3-d]pyrimidine-2,4(1H,3H)-dione,
3-Methyl-1-(1-methylethyl)-5-(1,3-thiazol-2-ylsulfonyl)-6-[2-(trifluoromethyl)benzyl]thieno[2,3-d]pyrimidine-2,4(1H,3H)-dione,
6-(9H-Fluoren-9-yl)-3-methyl-1-(2-methylpropyl)-5-(1H-1,2,4-triazol-5-ylsulfonyl)-thieno[2,3-d]pyrimidine-2,4(1H,3H)-dione,
6-(1H-Indol-3-ylcarbonyl)-3-methyl-1-(2-methylpropyl)-5-(1H-1,2,4-triazol-3-ylthio)-thieno[2,3-d]pyrimidine-2,4(1H,3H)-dione,
6-(Hydroxy-(1H-indol-3-yl)methyl)-3-methyl-1-(2-methylpropyl)-5-(1H-1,2,4-triazol-3-ylthio)-thieno[2,3-d]pyrimidine-2,4(1H,3H)-dione,
3-Methyl-1-(2-methylpropyl)-6-(4-quinolinylcarbonyl)-5-(1,3-thiazol-2-ylthio)-thieno[2,3-d]pyrimidine-2,4(1H,3H)-dione,
6-[Hydroxy-(4-quinolinyl)-methyl]-3-methyl-1-(2-methylpropyl)-5-(1,3-thiazol-2-ylthio)-thieno[2,3-d]pyrimidine-2,4(1H,3H)-dione,
6-(1-Benzothien-3-ylmethyl)-3-methyl-1-(2-methylpropyl)-5-(4H-1,2,4-triazol-3-ylthio)-thieno[2,3-d]pyrimidine-2,4(1H,3H)-dione,
and their pharmaceutically acceptable salts and solvates.

The present invention further provides a process for the preparation of a compound of formula (I) as defined above which comprises,
(a) when R represents C(O)Ar¹ and X represents a sulphur atom, reacting a compound of general formula 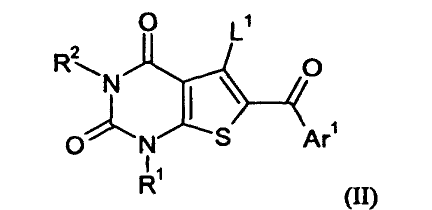 wherein L¹ represents a leaving group (e.g. a halogen atom such as bromine) and R¹, R² and Ar¹ are as defined in formula (I), with a compound of general formula (III), HS-Ar², wherein Ar² is as defined in formula (I), in the presence of a base (e.g. sodium hydride); or
(b) when R represents -C(R⁴)(R⁵)Ar¹, R⁴ represents a hydrogen atom, R⁵ represents hydroxyl and X represents a sulphur atom, reacting a corresponding compound of formula (I) in which R represents C(O)Ar¹ with a suitable reducing agent (e.g. sodium borohydride); or
(c) when R represents -C(R⁴)(R⁵)Ar¹, R⁴ represents a hydrogen atom, R⁵ represents a hydrogen atom and X represents a sulphur atom, reacting a corresponding compound of formula (I) in which R represents C(O)Ar¹ with a suitable reducing agent (e.g. BH₃.Me₂NH), in the presence of a Lewis acid (e.g. TiCl₄); or
(d) when R represents -C(R⁴)(R⁵)Ar¹ and R⁴ represents C₁₋₄ alkyl, reacting a corresponding compound of formula (I) in which R represents C(O)Ar¹ with a suitable Grignard reagent (e.g. C₁₋₄ alkylMgBr), followed by reaction with a dehydrating agent (e.g. methanesulphonyl chloride) in the presence of a base (e.g. triethylamine or 1,8-diazabicyclo[5.4.0]undec-7-ene, DBU), and then followed by a hydrogenation reaction; or
(e) when X represents SO or SO₂, reacting a corresponding compound of formula (I) in which X is a sulphur atom with a suitable oxidising agent; or
(f) when X represents CH(OH), reacting a compound of general formula 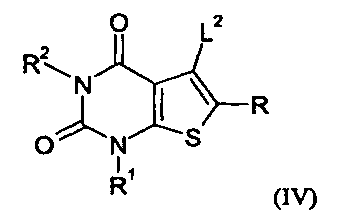 wherein L² represents a leaving group (e.g. a halogen atom such as bromine) and R, R¹ and R² are as defined in formula (I), with a suitable Grignard reagent (e.g. ethyl magnesium bromide) and then with a compound of general formula (V), Ar²CHO, wherein Ar² is as defined in formula (I); or
(g) when X represents C(O), reacting a corresponding compound of formula (I) in which X represents CH(OH) with a suitable oxidising agent (e.g. oxalyl chloride in dimethyl sulfoxide); or
(h) when X represents a sulphur atom, reacting a compound of formula (IV) as defined in (f) above with a suitable alkyl lithium compound or Grignard reagent and then with a compound of general formula (VI), Ar²-S-S-Ar², wherein Ar² is as defined in formula (I);
and optionally after (a), (b), (c), (d), (e), (f), (g) or (h) forming a pharmaceutically acceptable salt or solvate of the compound of formula (I) obtained.

Process (a) is conveniently carried out in an inert, aprotic solvent such as tetrahydrofuran at a temperature in the range from -20°C to 100°C, preferably from 10°C to 30°C.

Process (b) is conveniently carried out in an inert solvent such as ethanol at a temperature in the range from -20°C to 100°C, preferably from 10°C to 30°C.

Process (c) may be carried out in an inert, aprotic solvent such as dichloromethane at a temperature in the range from -20°C to 100°C, preferably from -10°C to 30°C.

Process (d), first step is performed in an inert solvent such as tetrahydrofuran at a temperature in the range from -50°C to 30°C, preferably from -20°C to 20°C. The resulting adduct is preferably contacted in the second step with the dehydrating agent and the base in an inert solvent such as dichloromethane at a temperature in the range from -50°C to 30°C, preferably from -10°C to 20°C. In the third step, the hydrogenation reaction is conveniently carried out using a palladium on carbon catalyst in ethanol at a hydrogen pressure of 1 to 5 x 10⁵ Nm⁻² and at a temperature in the range from 10°C to 30°C.

Process (e) is conveniently carried out in an inert, aprotic solvent such as dichloromethane at a temperature in the range from -20°C to 100°C, preferably from 0°C to 30°C. Suitable oxidising agents that may be used include 3-chloroperoxybenzoic acid or potassium peroxymonosulphate, commercially sold under the trade mark "OXONE".

In process (f), reaction with the Grignard reagent may be performed in an inert, aprotic solvent such as tetrahydrofuran at a temperature in the range from -20°C to 50°C, preferably from 0°C to 25°C. Subsequent reaction with the compound of general formula (VI) is conveniently carried out in the same solvent at a temperature in the range from -20°C to 100°C, preferably from 0°C to 25°C.

Process (g) is conveniently carried out using oxalyl chloride in a dimethyl sulphoxide solution at a temperature in the range from -78°C to -20°C, preferably from -78°C to -50°C.

Both steps of process (h) are conveniently performed in an inert, aprotic solvent such as tetrahydrofuran at a temperature in the range from -78°C to 50°C.

Compounds of formula (II) in which L¹ represents, for example, a bromine atom may be prepared by reacting a compound of general formula 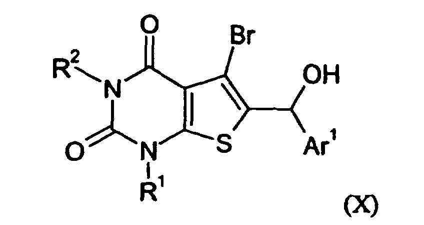 wherein R¹, R² and Ar¹ are as defined in formula (I), with an oxidising agent such as oxalyl chloride in dimethyl sulfoxide at a temperature in the range from -30°C to 30°C.

Compounds of formula (X) may be prepared by reacting a compound of general formula 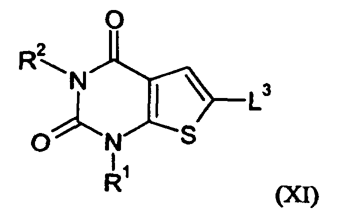 wherein L³ represents a halogen atom (e.g. bromine) and R¹ and R² are as defined in formula (I), with a lithium dialkylamide such as lithium diisopropylamide in an inert solvent (e.g. tetrahydrofuran) and at a temperature in the range from -78°C to 0°C, followed by reaction with a compound of general formula (XII), Ar¹CHO, at a temperature in the range from -78°C to 0°C.

Compounds of formula (XI) in which L³ represents, for example, a bromine atom may be prepared by reacting a compound of general formula 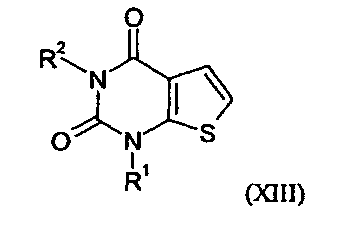 wherein R¹ and R² are as defined in formula (I), with bromine in an inert solvent (e.g. dichloromethane) at a temperature in the range from -20°C to 50°C.

Compounds of formula (IV) in which R represents -C(O)Ar¹ are equivalent to compounds of formula (II) and may be prepared as described above.

Compounds of formula (IV) in which R represents -C(R⁴)(R⁵)Ar¹, R⁴ represents a hydrogen atom and R⁵ represents hydroxyl are equivalent to compounds of formula (X) and may be prepared as described above.

Compounds of formula (IV) in which R represents -C(R⁴)(R⁵)Ar¹, R⁴ represents a hydrogen atom and R⁵ represents a hydrogen atom may be prepared by reacting a compound of formula (X) with trifluoroacetic acid/triethylsilane at a temperature in the range from -20°C to 50°C, optionally in the presence of an inert solvent such as dichloromethane.

Compounds of formula (IV) in which R represents -C(R⁴)(R⁵)Ar¹ and R⁴ represents C₁₋₄ alkyl, may be prepared by reacting a compound of formula (II) by a process analogous to process (d) above.

Compounds of formula (IV) in which R represents Ar³ may be prepared by reacting a compound of formula (XI) with a lithium dialkylamide such as lithium diisopropylamide in an inert solvent (e.g. tetrahydrofuran) at a temperature in the range from -78°C to 0°C, followed by reaction with acenaphthenone, indanone or fluorenone at a temperature in the range from -78°C to 0°C in the same solvent, and then reduction of the adduct using triethylsilane in the presence of a strong acid such as trifluoroacetic acid either in the absence of solvent or in an inert solvent (e.g. dichloromethane) at a temperature in the range from 0°C to 30°C.

Compounds of formulae (III), (V), (VI), (XII) and (XIII) are either commercially available, are well known in the literature or may be prepared easily using known techniques.

It will be appreciated by those skilled in the art that in the processes of the present invention certain functional groups such as hydroxyl or amino groups in the starting reagents or intermediate compounds may need to be protected by protecting groups. Thus, the preparation of the compounds of formula (I) may involve, at an appropriate stage, the removal of one or more protecting groups.

The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley―Interscience (1991).

The compounds of formula (I) above may be converted to a pharmaceutically acceptable salt or solvate thereof, preferably an acid addition salt such as a hydrochloride, hydrobromide, phosphate, acetate, fumarate, maleate, tartrate, citrate, oxalate, methanesulfonate or p-toluenesulfonate, or an alkali metal salt such as a sodium or potassium salt.

Certain compounds of formula (I) are capable of existing in stereoisomeric forms. It will be understood that the invention encompasses all geometric and optical isomers, including atropisomers, of the compounds of formula (I) and mixtures thereof including racemates. Tautomers and mixtures thereof also form an aspect of the present invention.

Isomers may be resolved or separated by conventional techniques, e.g. chromatography or fractional crystallisation. Enantiomers may be isolated by separation of a racemic or other mixture of the compounds using conventional techniques (e.g. chiral HPLC). Alternatively the desired optical isomers may be made by reaction of the appropriate optically active starting materials under conditions which will not cause racemisation, or by derivatisation, for example with a homochiral acid followed by separation of the diastereomeric derivatives by conventional means (e.g. HPLC, chromatography over silica) or may be made with achiral starting materials and chiral reagents. All stereoisomers are included within the scope of the invention.

The compounds of the invention may be isolated from their reaction mixtures using conventional techniques.

The compounds of the invention are useful because they possess pharmacological activity in human and non-human animals. They are therefore indicated as pharmaceuticals for use in the (prophylactic) treatment of autoimmune, inflammatory, proliferative and hyperproliferative diseases and immunologically-mediated diseases including rejection of transplanted organs or tissues and Acquired Immunodeficiency Syndrome (AIDS).

Examples of these conditions are:
(1) (the respiratory tract) obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) (bone and joints) rheumatoid arthritis, seronegative spondyloarthropathies (including ankylosing spondylitis, psoriatic arthritis and Reiter's disease), Behcet's disease, Sjogren's syndrome and systemic sclerosis;
(3) (skin) psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) (gastrointestinal tract) Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) (other tissues and systemic disease) multiple sclerosis, atherosclerosis, Acquired Immunodeficiency Syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura;
(6) (allograft rejection) acute and chronic following, for example, transplantation of kidney, heart, liver, lung, bone marrow, skin and cornea; and chronic graft versus host disease; and
(7) cancer.

Accordingly, the present invention provides a compound of formula (I) or a pharmaceutically acceptable salt or solvate thereof as hereinbefore defined for use in therapy.

In another aspect, the invention provides the use of a compound of formula (I) or a pharmaceutically acceptable salt or solvate thereof as hereinbefore defined in the manufacture of a medicament for use in therapy.

In the context of the present specification, the term "therapy" also includes "prophylaxis" unless there are specific indications to the contrary. The terms "therapeutic" and "therapeutically" should be construed accordingly.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

The invention further provides a method of effecting immunosuppression (e.g. in the treatment of allograft rejection) which comprises administering to a patient a therapeutically effective amount of a compound of formula (I) or a pharmaceutically acceptable salt or solvate thereof as hereinbefore defined.

The invention still further provides a method of treating, or reducing the risk of, an obstructive airways disease (e.g. asthma or COPD) in a patient suffering from, or at risk of, said disease, which comprises administering to the patient a therapeutically effective amount of a compound of formula (I) or a pharmaceutically acceptable salt or solvate thereof as hereinbefore defined.

For the above-mentioned therapeutic uses the dosage administered will, of course, vary with the compound employed, the mode of administration, the treatment desired and the disorder indicated. However, in general, for effecting immunosuppression, the daily dosage of the compound of formula (I) will be in the range from 0.1 mg/kg, preferably from 0.3 mg/kg, more preferably from 0.5 mg/kg and still more preferably from 1 mg/kg up to and including 30 mg/kg. For the treatment of obstructive airways diseases, the daily dosage of the compound of formula (I) will typically be in the range from 0.001 mg/kg to 30 mg/kg.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably less than 80 %w, e.g. from 0.10 to 70 %w, and even more preferably less than 50 %w, of active ingredient, all percentages by weight being based on total composition.

Thus, the present invention also provides a pharmaceutical composition comprising a compound of formula (I) or a pharmaceutically acceptable salt or solvate thereof as hereinbefore defined, in association with a pharmaceutically acceptable adjuvant, diluent or carrier.

The invention further provides a process for the preparation of a pharmaceutical composition of the invention which comprises mixing a compound of formula (1) or a pharmaceutically acceptable salt or solvate thereof as hereinbefore defined, with a pharmaceutically acceptable adjuvant, diluent or carrier.

The pharmaceutical composition of the invention may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally.

The invention will now be further explained by reference to the following illustrative examples.

### Preparation 1

### a) 5-Bromo-6-{hydroxy[2-(trifluoromethyl)phenyl]methyl}-1-isobutyl-3-methyl-thieno[2,3-d]pyrimidine-2,4(1H,3H)-dione

1-Isobutyl-3-methyl-thieno[2,3-d]pyrimidine-2,4(1H,3H)-dione (20g) was dissolved in DCM (100 mls) and cooled to 0°C before bromine (4.94 mls) was added dropwise. Ten minutes after full addition the mixture was concentrated in vacuo, redissolved in ethyl acetate and washed with a 50% aqueous sodium hydrogen bicarbonate followed by sodium thiosulfate and then brine. The ethyl acetate was separated, dried over magnesium sulfate, filtered and concentrated in vacuo to a pale yellow solid. This solid was stored under a high vacuum for 1 hour before being dissolved in tetrahydrofuran (THF) (160 mls) and cooled to -78°C. To this solution was slowly added lithium diisopropylamide (LDA) (126 mls of 1M solution in isohexanes/THF). After complete addition the reaction was stirred for 30 minutes before 2-trifluoromethyl benzaldehyde (16.61 mls) was added neat in 1 aliquot. The resultant solution was allowed to stir 2 hrs before 50mls of water was added and the cooling bath removed. Once the reaction mixture had reached room temperature it was concentrated in vacuo and partitioned between ethyl acetate and water. The ethyl acetate was collected, dried over magnesium sulfate, filtered and concentrated in vacuo to a dark oil. The oil was purified via silica chromatography, eluting with 4:1 isohexane:ethyl acetate to yield 33.47g (81%) of the subtitle compound.
MS: [M-OH]⁺ 474

### b) 5-Bromo-1-isobutyl-3-methyl-6-[2-(trifluoromethyl)benzyl]-thieno[2,3-d]pyrimidine-2,4(1H,3H)-dione

5-Bromo-6-{hydroxy[2-(trifluoromethyl)phenyl]methyl}-1-isobutyl-3-methyl-thieno[2,3-d]pyrimidine-2,4(1H,3H)-dione (33.47 g) was dissolved in triethyl silane (100 mls) and cooled to 0°C before trifluoroacetic acid (100 mls) was added dropwise. After complete addition the resultant thick suspension was diluted by the addition of dichloromethane (DCM) (100 mls) and the reaction was stirred at room temperature for 18 hours. The reaction mixture was then concentrated in vacuo and partitioned between DCM and 50% aqueous sodium hydrogen carbonate. The organic layer was collected, dried over magnesium sulfate, filtered and concentrated in vacuo. The resultant off white solid was stirred vigorously with isohexane for 1 hour before being collected by filtration to yield 27.286g (84.5%) of the subtitle compound.
MS: [M+H]⁺ 476

### Example 1

### 1-Isobutyl-3-methyl-5-[(1-methyl-1H-pyrrol-2-yl)carbonyl]-6-[2-(trifluoromethyl)benzyl]-thieno[2,3-d]pyrimidine-2,4(1H,3H)-dione

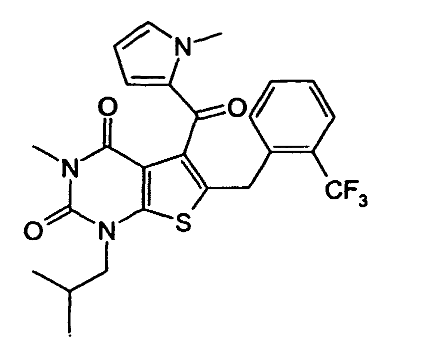

5-Bromo-1-isobutyl-3-methyl-6-[2-(trifluoromethyl)benzyl]-thieno[2,3-d]pyrimidine-2,4(1H,3H)-dione (100 mg) was dissolved in THF(1 ml) under nitrogen to which ethyl magnesium bromide (1M, 210 µl) was added in 1 aliquot. After 15 minutes 1-methyl-1H-pyrrole-2-carbaldehyde (1M in THF, 210 µl) was added and the reaction was left for 1 hour. Water (2 ml) was added and the reaction was stored under high vacuum. Once all of the THF had evaporated, the water was extracted twice with DCM. This DCM solution was then added to a preformed mixture of oxalyl chloride(50 µl) and dimethyl sulphoxide (DMSO) (100 µl) in DCM (1 ml) at -78°C. After 10 minutes triethylamine (260 µl) was added and the reaction was stirred for 30 minutes before being allowed to warm to room temperature. Once at room temperature the reaction was washed with water (5 ml) and evaporated to dryness. The residue was purified by silica chromatography, eluting with a 0 to 75% gradient of isohexane in diethyl ether to yield the title compound.
MS: [M+H]⁺ 504
¹H NMR (d₆-DMSO) δ 0.87 (6H, d), 2.09-2.13 (1H, m), 3.12 (3H, s), 3.21 (3H,s), 3.66 (2H, d), 4.23 (2H, d), 6.31 (1H, d), 6.53 (1H, s), 7.22 (1H, d), 7.40 (1H, d), 7.47 (1H, t), 7.65 (1H, t), 7.72 (1H, d)

### Example 2

### 3-Methyl-1-(2-methylpropyl)-5-(1H-1,2,4-triazol-3-ylthio)-6-[2-(trifluoromethyl)benzoyl]-thieno[2,3-d]pyrimidine-2,4(1H,3H)-dione

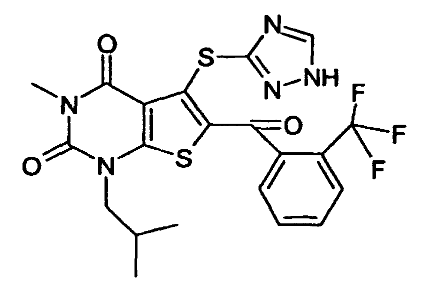

### a) 5-Bromo-3-methyl-1-(2-methylpropyl)-6-[2-(trifluoromethyl)benzoyl]-thieno[2,3-d]pyrimidine-2,4(1H,3H)-dione

A solution of 5-bromo-6-[hydroxy[2-(trifluoromethyl)phenyl]methyl]-3-methyl-1-(2-methylpropyl)-thieno[2,3-d]pyrimidine-2,4(1H,3H)-dione, (6.44 g) (prepared as described in preparation 1 part a)), 4-methyl morpholine-N-oxide (2.3 g), powdered 4Å sieves (6.55 g) and tetra-n-propylammonium perruthenate (0.23 g) in dry dichloromethane (120 ml) was stirred for 1 hour at room temperature. The suspension was filtered through Celite and the filtrate was concentrated under reduced pressure. The residue was purified by column chromatography, eluting with dichloromethane, to give the sub-title compound as a pale cream foam (6.08 g).
MS (APCI) 490.9{M+H]+

### b) 3-Methyl-1-(2-methylpropyl)-5-(1H-1,2,4-triazol-3-ylthio)-6-[2-(trifluoromethyl)benzoyl]-thieno[2,3-d]pyrimidine-2,4(1H,3H)-dione

3-Mercapto-1,2,4-triazole (0.45 g) in dry tetrahydrofuran (10 ml) was added dropwise to a suspension of sodium hydride (0.196 g, 60% oil dispersion) in dry tetrahydrofuran (30 ml) at room temperature under nitrogen. After 15 minutes, a solution of 5-bromo-3-methyl-1-(2-methylpropyl)-6-[2-(trifluoromethyl)benzoyl]-thieno[2,3-d]pyrimidine-2,4(1H,3H)-dione (2.0 g) in dry tetrahydrofuran (20 ml) was added dropwise and the reaction was stirred for 24 hours at room temperature. The solution was poured into water and extracted with ethyl acetate. The combined extracts were dried over magnesium sulfate, filtered and concentrated under reduced pressure. The residue was purified by flash silica chromatography eluting with 4% ethanol in dichloromethane to give the title compound as a yellow foam (2.0 g).
MS (APCI) 509.9[M+H]+
¹H NMR(CDCl₃) δ 1.03 (6H,d), 2.34 (1H,septet), 3.30 (3H,s), 3.86 (2H,d), 7.49-7.60 (3H,m), 7.75 (1H,d), 7.98 (1H,s).

### Example 3

### 3-Methyl-1-(2-methylpropyl)-5-(1H-1,2,4-triazol-3-ylthio)-6-[[2-(trifluoromethyl)phenyl]methyl]-thieno[2,3-d]pyrimidine-2,4(1H,3H)-dione

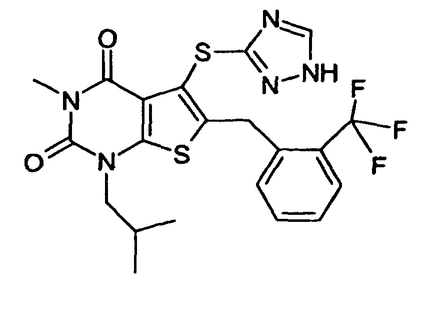

Titanium tetrachloride in dichloromethane (1.0M, 13.74 ml) was added to a solution of 3-methyl-1-(2-methylpropyl)-5-(1H-1,2,4-triazol-3-ylthio)-6-[2-(trifluoromethyl)benzoyl]-thieno[2,3-d]pyrimidine-2,4(1H,3H)-dione (2.0 g, as prepared in Example 2) in dry dichloromethane (80 ml) at 0°C. Dimethylamine-borane complex in dry dichoromethane (20 ml) was added dropwise. The solution was allowed to warm to room temperature and stirred for 3 hours. Hydrochloric acid (2.0M, 10 ml) was added to the reaction mixture and stirring continued for 5 minutes. The solution was extracted with dichloromethane and the combined extracts were washed with brine, dried over anhydrous magnesium sulfate, filtered and concentrated under reduced pressure. The residue was purified by flash silica chromatography eluting with a gradient of 1-6% ethanol in dichloromethane to give a solid which was recrystallised from acetonitrile to give the title compound as a white solid (0.69g).
m.p. 186-187 °C
MS (APCI) 496[M+H]+
¹H NMR (CDCl₃) δ 0.92 (6H,d), 2.19 (1H,septet), 3.45 (3H,s), 3.70 (2H,d), 4.69 (2H,s), 7.30 (1H,d), 7.37 (1H,t), 7.47 (1H,t), 7.69 (1H,d), 8.02 (1H,s).

### Example 4

### 3-Methyl-1-(2-methylpropyl)-5-(1H-1,2,4-triazol-3-ylsulfonyl)-6-[[2-(trifluoromethyl)phenyl]methyl]-thieno[2,3-d]pyrimidine-2,4(1H,3H)-dione

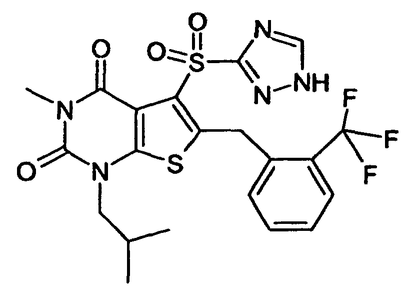

3-Chloroperoxybenzoic acid (0.22 g, 57-86%) was added to a suspension of 3-methyl-1-(2-methylpropyl)-5-(1H-1,2,4-triazol-3-ylthio)-6-[[2-(trifluoromethyl)phenyl]methyl]-thieno[2,3-d]pyrimidine-2,4(1H,3H)-dione (0.16 g, prepared as in Example 3) in dry dichloromethane (50 ml) and stirred at room temperature for 2 hours. The reaction mixture was diluted with ethyl acetate and washed with saturated sodium metabisulfite solution, saturated sodium bicarbonate solution, then brine. The organic layer was dried over anhydrous magnesium sulfate, filtered and concentrated under reduced pressure. The residue was purified by flash silica chromatography eluting with a gradient of 0-5% ethanol in dichloromethane to give the title compound as a white foam (0.092 g).
MS (ES+) 528 {M+H]+
¹H NMR (CDCl₃) δ 0.91 (6H,d), 2.16 (1H,septet), 3.26 (3H,s), 3.70 (2H,d), 5.03 (2H,s), 7.26-7.27 (1H,m), 7.44 (1H,t), 7.52-7.58 (2H,m), 7.73 (1H,d), 8.41 (1H,br.s).

By the method of Example 2), step b the following compounds were prepared:

### Example 5

### 3-Methyl-1-(2-methylpropyl)-5-(2-thiazolylthio)-6-[2-(trifluoromethyl)benzoyl]-thieno[2,3-d]pyrimidine-2,4(1H,3H)-dione

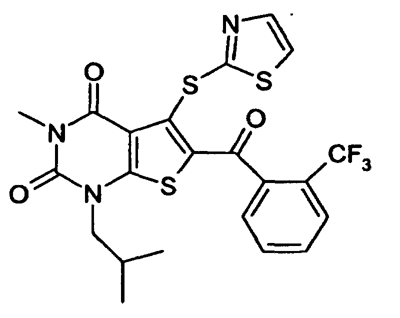

Prepared using 2-mercaptothiazole and purified by flash silica chromatography eluting with a gradient of 0-60% ethyl acetate in isohexane.
MS (APCI) 526[M+H]+
¹H NMR (CDCl₃) δ 1.04 (6H,d), 2.37 (1H, septet), 3.29 (3H,s), 3.87 (2H,d), 7.12 (1H,d), 7.40-7.44 (2H,m), 7.52-7.60 (2H,m), 7.74 (1H,d).

### Example 6

### 3-Methyl-1-(2-methylpropyl)-5-[[5-(methylthio)-1,3,4-thiadiazol-2-yl]thio]-6-[2-(trifluoromethyl)benzoyl]-thieno[2,3-d]pyrimidine-2,4(1H,3H)-dione

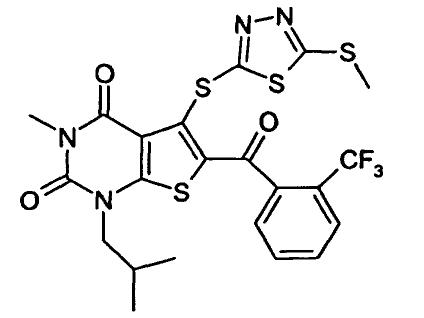

Prepared using 5-methylthio-1,3,4-thiadiazole-2-thiol and purified by flash silica chromatography eluting with a gradient of 0-60% ethyl acetate in isohexane.
MS (APCI) 573[M+H]+
¹H NMR (CDCl₃) δ 1.04 (6H,d), 2.36 (1H, septet), 2.72 (3H,s), 3.30 (3H,s), 3.86 (2H,d), 7.46-7.47 (2H,m), 7.55-7.60 (1H,m), 7.77 (1H,br.d).

### Example 7

### 3-Methyl-5-[(4-methyl-2-oxazolyl)thio]-1-(2-methylpropyl)-6-[2-(trifluoromethyl)benzoyl]-thieno[2,3-d]pyrimidine-2,4(1H,3H)-dione

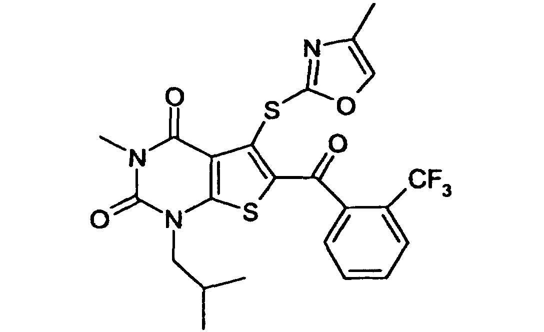

Prepared using 4-methyl-2-oxazolethiol and purified by flash silica chromatography eluting with 0-60% ethyl acetate in isohexane.
MS (APCI) 524[M+H]+
¹H NMR (CDCl₃) δ 1.0 (6H,d), 2.07 (3H,s), 2.35 (1H,septet), 3.30 (3H,s), 3.86 (2H,d), 7.26-7.27 (1H,m), 7.43 (1H,d), 7.51 (1H,t), 7.59 (1H,t), 7.75 (1H,d).

### Example 8

### 3-Methyl-1-(2-methylpropyl)-5-[(5-methyl-1H-1,2,4-triazol-3-yl)thio]-6-[2-(trifluoromethyl)benzoyl]-thieno[2,3-d]pyrimidine-2,4(1H,3H)-dione

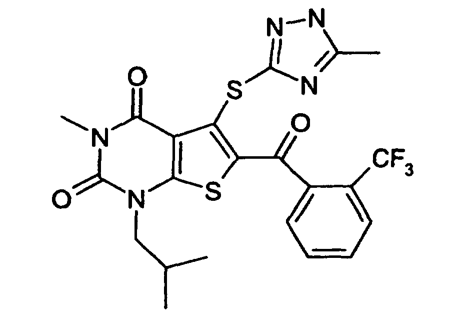

Prepared using 5-methyl-1H-1,2,4-triazole-3-thiol and purified by flash silica chromatography eluting with 10% ethanol in ethyl acetate.
m.p. 220-222 °C
MS (APCI) 524{M+H]+
¹H NMR(CDCl₃) δ 0.98 (6H,d), 2.35 (1H,septet), 2.36 (3H,s), 3.30 (3H,s), 3.85 (2H,d), 7.50-7.60 (3H,m), 7.73 (1H,d).

### Example 9

### 3-Methyl-1-(2-methylpropyl)-5-[(5-hydroxy-4-(1-methylethyl)-1H-1,2,4-triazol-3-yl)thio]-6-[2-(trifluoromethyl)benzoyl]-thieno[2,3-d]pyrimidine-2,4(1H,3H)-dione

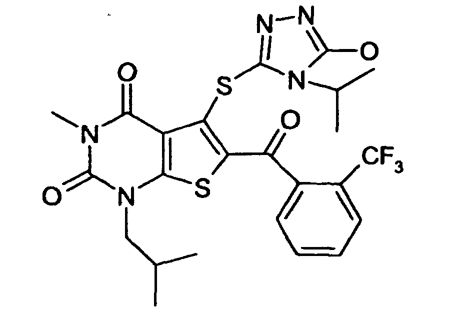

Prepared using 5-hydroxy-4-isopropyl-4H-1,2,4-triazole-3-thiol and purified by flash silica chromatography eluting with a gradient of 0-2% ethanol in dichloromethane.
MS (APCI) 568 {M+H]+
¹H NMR(CDCl₃) δ 0.86 (6H,d), 1.37 (6H,d), 2.34 (1H,septet), 3.31 (3H,s), 3.85 (2H,d), 4.17 (1H,quintet), 7.47-7.62 (3H,m), 7.76 (1H,d), 9.07 (1H,br.s).

### Example 10

### 3-Methyl-1-(2-methylpropyl)-5-[(5-methyl-1,3,4-thiadiazol-2-yl)thio]-6-[2-(trifluoromethyl)benzoyl]-thieno[2,3-d]pyrimidine-2,4(1H,3H)-dione

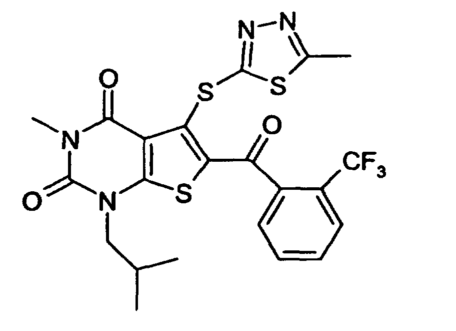

Prepared using 2-mercapto-5-methyl-1,3,4-thiadiazole and purified by flash silica chromatography eluting with a gradient of 20-100% ethyl acetate in isohexane.
MS (APCI) 541 [M+H]+
¹H NMR (CDCl₃) δ 1.04 (6H,d), 2.36 (1H,septet), 2.69 (3H,s), 3.28 (3H,s), 3.87 (2H,d), 7.40-7.49 (2H,m), 7.53-7.60 (1H,m), 7.76 (1H,d).

### Example 11

### 5-(1H-Imidazol-2-ylthio)-3-methyl-1-(2-methylpropyl)-6-[2-(trifluoromethyl)benzoyl]-thieno[2,3-d]pyrimidine-2,4(1H,3H)-dione

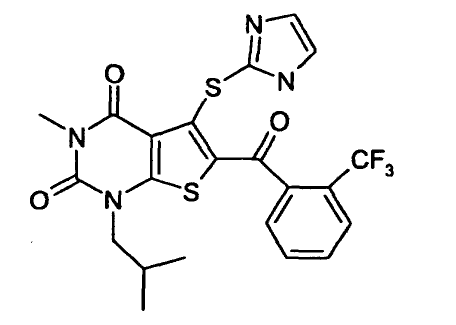

Prepared using 2-mercaptoimidazole and purified by flash silica chormatography with a gradient for 0-6% ethanol in dichloromethane.
m.p. 123-127 °C
MS (APCI) 509.09[M+H]+
¹H NMR (CDCl₃) δ 1.01 (6H,d), 2.32 (1H,septet), 3.39 (3H,s), 3.85 (2H,d), 6.99-7.03 (2H,m), 7.60-7.65 (1H,m), 7.70-7.71 (2H,m), 7.77 (1H,d).

### Example 12

### 5-[[4-(1,1-Dimethylethyl)-4H-1,2,4-triazol-3-yl]thio]-3-methyl-1-(2-methylpropyl)-6-[2-(trifluoromethyl)benzoyl]-thieno[2,3-d]pyrimidine-2,4(1H,3H)-dione

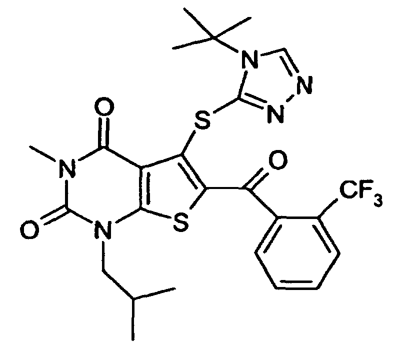

Prepared using 4-(1,1-dimethylethyl)-4H-1,2,4-triazole-3-thiol and purified by flash silica chromatography eluting with a gradient of 0-5% ethanol in dichloromethane.
MS (APCI) 566[M+H]+
¹H NMR (CDCl₃) δ 1.03 (6H,d), 1.55 (9H,d), 2.34 (1H,septet), 3.23 (3H,s), 3.85 (2H,d), 7.37 (1H,d), 7.47 (1H,t), 7.58 (1H,d), 7.73 (1H,d), 8.10 (1H,s).

### Example 13

### 5-[(5-Amino-1,3,4-thiadiazol-2-yl)thio]-3-methyl-1-(2-methylpropyl)-6-[2-(trifluoromethyl)benzoyl]-thieno[2,3-d]pyrimidine-2,4(1H,3H)-dione

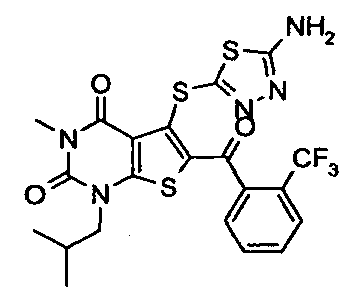

Prepared using 2-amino-5-mercapto-1,3,4-thiadiazole and purified by column chromatography over silica, eluting with ethyl acetate then by re-crystallisation from ethyl acetate/isohexane.
MS (+ve APCI) 542 ((M+H)+)
¹H NMR (CDCl₃) δ 1.03 (6H, d), 2.29-2.41 (1H, m), 3.32 (3H, s), 3.86 (2H, d), 4.97 (2H, s, br), 7.26-7.62 (3H, m), 7.76 (1H, d).

### Example 14

### 3-Methyl-1-(2-methylpropyl)-5-(1,3,4-thiadiazol-2-ylthio)-6-[2-(trifluoromethyl)benzoyl]-thieno[2,3-d]pyrimidine-2,4(1H,3H)-dione

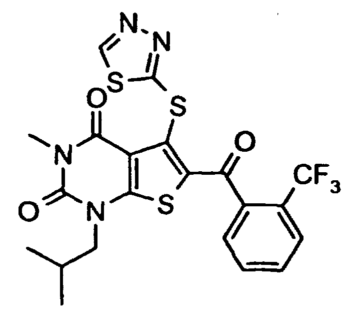

Prepared using 2-mercapto-1,3,4-thiadiazole and recrystallised from ethyl acetate/isohexane.
MS (+ve APCI) 527 ((M+H)+)
¹H NMR (DMSO-D6 ) δ 0.98 (6H, d), 2.20-2.32 (1H, m), 3.12 (3H, s), 3.86 (2H, d), 0.76-7.63 (2H, m), 7.72-7.76 (2H, m), 7.89 (1H, d), 9.44 (1H, s).

### Example 15

### 6-[Hydroxy[2-(trifluoromethyl)phenyl]methyl]-5-(1H-imidazol-2-ylthio)-3-methyl-1-(2-methylpropyl)-thieno[2,3-d]pyrimidine-2,4(1H,3H)-dione

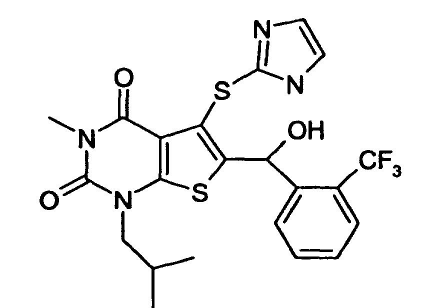

Sodium borohydride (0.008 g) was added to a solution of the product of example 5 viii) (0.10 g,) in dry ethanol and stirred at room temperature under nitrogen for 24 hours. The solution was concentrated under reduced pressure and the residue was partitioned between ethyl acetate and water. The organic layer was dried over anhydrous magnesium sulfate, filtered and concentrated under reduced pressure. The residue was purified by flash silica chromatography eluting with 10% ethanol in dichloromethane to give the title compound as a white foam (0.050 g).
MS (APCI) 511 [M+H]+
¹H NMR (CDCl₃) δ 0.86 (6H,dd), 2.11 (2H,m), 3.39 (3H,s), 3.53 (1H,dd), 3.71 (1H,dd), 7.05 (2H,s), 7.26 (1H,s), 7.50 (1H,t), 7.68-7.75 (2H,m), 8.23 (1H,d).

### Example 16

### 6-[Hydroxy[2-(trifluoromethyl)phenyl]methyl]-3-methyl-1-(2-methylpropyl)-5-(1H-1,2,4-triazol-3-ylthio)-thieno[2,3-d]pyrimidine-2,4(1H,3H)-dione

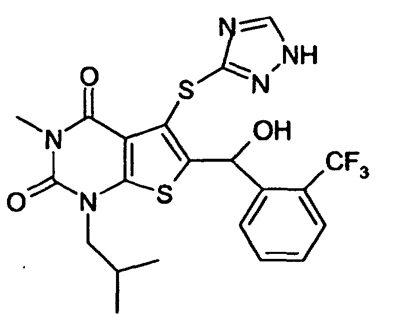

Prepared from the compound of Example 2 by the method of Example 15.
MS (APCI) 512 [M+H]+
¹H NMR (CDCl₃) δ 0.89 (6H,dd), 2.14 (1H,septet), 3.39 (3H,s), 3.57 (1H,dd), 3.74 (1H,dd), 7.18 (1H,s), 7.52 (1H,t), 7.69-7.76 (2H,m), 8.11 (1H,s), 8.23 (1H,br.d).

By the method of Example 3 were prepared the following compounds:

### Example 17

### 3-Methyl-1-(2-methylpropyl)-5-(2-thiazolylthio)-6-[[2-(trifluoromethyl)phenyl]methyl]-thieno[2,3-d]pyrimidine-2,4(1H,3H)-dione

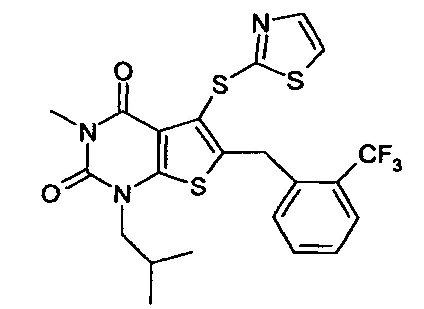

Prepared from the compound of Example 5.
m.p. 141-144 °C
MS (ES+) 512{M+H]+
¹H NMR (DMSO) δ 0.87 (6H,dd), 2.11-2.16 (1H,m), 3.18 (3H,d), 3.71 (2H,d), 4.53 (2H,s), 7.40 (1H,d), 7.51 (1H,t), 7.54-7.57 (1H,m), 7.62-7.68 (2H,m), 7.77 (1H,d).

### Example 18

### 3-Methyl-1-(2-methylpropyl)-5-[(5-methyl-1H-1,2,4-triazol-3-yl)thio]-6-[[2-(trifluoromethyl)phenyl]methyl]-thieno[2,3-d]pyrimidine-2,4(1H,3H)-dione dihydrochloride

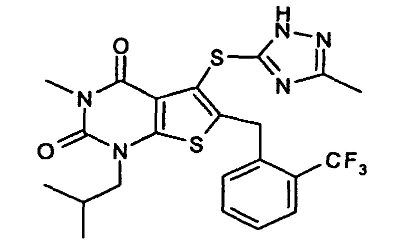

Prepared from the compound of Example 8.
m.p. 154 °C
MS (APCI) 510[M+H]+
¹H NMR (DMSO) δ 0.86 (6H,d), 2.12 (1H,septet), 2.25 (3H,s), 3.18 (3H,s), 3.53 (1H,br.s), 3.68 (2H,d), 4.42 (2H,s), 7.36 (1H,d), 7.48 (1H,t), 7.59 (1H,t), 7.75 (1H,d).

### Example 19

### 5-(1H-Imidazol-2-ylthio)-3-methyl-1-(2-methylpropyl)-6-[[2-(trifluoromethyl)phenyl]methyl]-thieno[2,3-d]pyrimidine-2,4(1H,3H)-dione

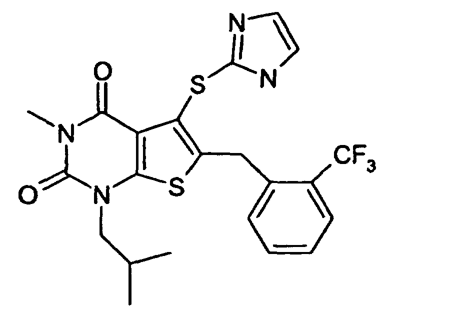

Prepared from the compound of Example 11.
m.p. 221-225 °C
MS (ES+) 495[M+H]
¹H NMR (CDCl₃) δ 0.90 (6H,d), 2.14-2.23 (1H,m), 3.49 (3H,s), 3.69 (2H,d), 4.78 (2H,s), 7.11 (2H,d), 7.35-7.38 (2H,m), 7.43-7.48 (1H,m), 7.68 (1H,d), 11.34 (1H,br.s).

### Example 20

### 3-Methyl-1-(2-methylpropyl)-5-(1,3,4-thiadiazol-2-ylthio)-6-[2-(trifluoromethyl)benzyl]-thieno[2,3-d]pyrimidine-2,4(1H,3H)-dione

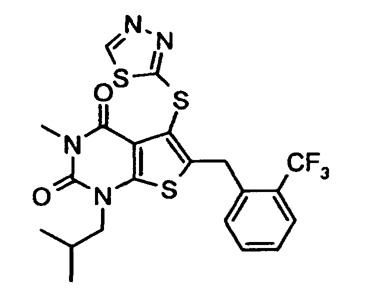

Prepared from the compound of Example 14.
m.p. 169-170°C
MS (+ve APCI) 513 ((M+H)+)
¹H NMR (DMSO-D6 ) δ 0.89 (6H, d), 2.08-2.12 (1H, m), 3.17 (3H, s), 3.71 (2H, d), 4.55 (2H, s), 7.40 (1H, d), 7.51 (1H, d), 7.64 (1H, t), 7.77 (1H, d), 9.45 (1H, s).

### Example 21

### 5-[(5-Amino-1,3,4-thiadiazol-2-yl)thio]-3-methyl-1-(2-methylpropyl)-6-[2-(trifluoromethyl)benzyl]-thieno[2,3-d]pyrimidine-2,4(1H,3H)-dione

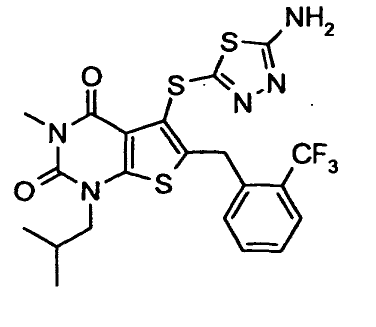

Prepared from the compound of Example 13.
m.p. 236-7°C
MS (+ve APCI) 528 ((M+H)+)
¹H NMR (DMSO-D6 ) δ 0.86 (6H, d), 2.05-2.14 (1H, m), 3.23 (3H, s), 3.68 (2H, d), 4.56 (2H, s), 7.24 (2H, s), 7.37 (1H, d), 7.52 (1H, t), 7.64 (1H, t), 7.78 (1H, d).
Using the method of Example 4, the following compounds were prepared.

### Example 22

### 3-Methyl-1-(2-methylpropyl)-5-[(5-methyl-1H-1,2,4-triazol-3-yl)sulfonyl]-6-[[2-(trifluoromethyl)phenyl]methyl]-thieno[2,3-d]pyrimidine-2,4(1H,3H)-dione

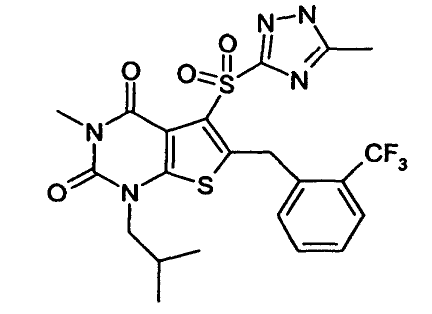

Prepared using the compound of Example 18 and purified by flash silica chromatography eluting with a gradient of 2-4% ethanol in dichloromethane.
MS(APCI) 542[M+H]+
¹H NMR (CDCl₃) δ 0.92 (6H,d), 1 61 (1H,br.s.), 2.17 (1H,septet), 2.58 (3H,s), 3.28 (3H,s), 3.68 (2H,d), 5.02 (2H,s), 7.43-7.46 (1H,m), 7.53-7.60 (2H,m), 7.72 (1H,d).

### Example 23

### 5-(1H-Imidazol-2-ylsulfonyl)-3-methyl-1-(2-methylpropyl)-6-[[2-(trifluoromethyl)phenyl]methyl]-thieno[2,3-d]pyrimidine-2,4(1H,3H)-dione

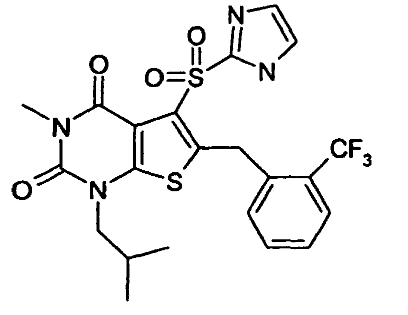

Prepared using the compound of Example 19 and purified by flash silica chromatography eluting with ethyl acetate:isohexane (1:1).
MS(APCI) 527[M+H]+
¹H NMR (CDCl₃) δ 0.89 (6H,d), 2.12 (1H,septet), 3.40 (3H,s), 3.63 (2H,d), 5.03 (2H,s), 7.28-7.31 (2H,m), 7.40-7.57 (3H,m), 7.72 (1H,d), 11.74 (1H,br.s).

### Example 24

### 6-[Hydroxy[2-(trifluoromethyl)phenyl]methyl]-3-methyl-1-(2-methylpropyl)-5-(1H-1,2,4-triazol-3-ylsulfonyl)-thieno[2,3-d]pyrimidine-2,4(1H,3H)-dione

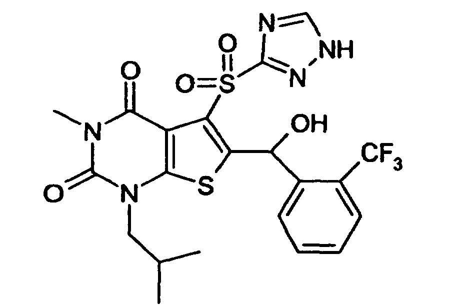

Prepared using the compound of Example 16 and purified by flash silica chromatography eluting with 4-10% ethanol in dichloromethane.
m.p. 198-202 °C
MS(APCI) 544[M+H]+
¹H NMR (CDCl₃ + DMSO) δ 0.90-0.97 (6H,m), 2.20 (1H,septet), 2.57-2.60 (1H,m), 3.22 (3H,s), 3.65-3.82 (2H,m), 6.98 (1H,s), 7.48 (1H,t), 7.59 (1H,t), 7.74 (2H,d), 8.29 (1H,s).

### Example 25

### 3-Methyl-1-(2-methylpropyl)-5-(1,3-thiazol-2-ylsulfonyl)-6-[2-(trifluoromethyl)benzyl]-thieno[2,3-d]pyrimidine-2,4(1H,3H)-dione

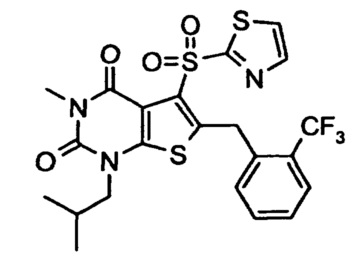

Prepared using the compound of Example 17 and purified by normal-phase preparative HPLC with gradient: dichloromethane/ethanol elution, followed by recrystallisation from ethyl acetate/iso-hexane.
MS (+ve APCI) 544 ((M+H)+)
¹H NMR (CDCl₃) δ 0.91 (6H, d), 2.10-2.21 (1H, m), 3.27 (3H, s), 3.65 (2H, d), 5.03 (2H, s), 7.42-7.48 (1H, m), 7.53-7.59 (2H, m), 7.22-7.75 (2H, m), 7.91 (1H, d).

### Example 26

### 3-Methyl-1-(2-methylpropyl)-5-(1,3,4-thiadiazol-2-ylsulfonyl)-6-[2-(trifluoromethyl)benzyl]-thieno[2,3-d]pyrimidine-2,4(1H,3H)-dione

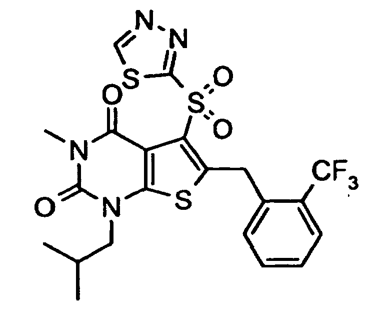

Prepared using the compound of Example 20 and purified by column chromatography over silica, eluting with ethyl acetate/isohexane (2:1), and then recrystallised from ethyl acetate/isohexane.
m.p. 148-149°C
MS (+ve APCI) 545 ((M+H)+)
¹H NMR (DMSO-D6 ) δ 0.85 (6H, d), 2.00-2.12 (1H, m), 3.08 (3H, s), 3.65 (2H, d), 4.96 (2H, s), 7.56 (1H, d), 7.59 (1H, t), 7.73 (1H, t), 7.82 (1H, d), 9.94 (1H, s).

### Example 27

### 5-[(3-Bromo-1H-1,2,4-triazol-5-yl)thio]-3-methyl-1-(2-methylpropyl)-6-[2-(trifluoromethyl)phenylmethyl]-thieno[2,3-d]pyrimidine-2,4(1H,3H)-dione

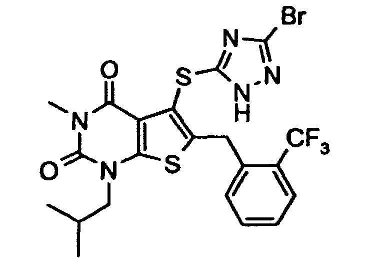

### a) 3,5-Dibromo-1-(4-methoxyphenylmethyl)-1H-1,2,4-triazole

4-Methoxyphenylmethyl chloride (4.47ml) was added to a stirred solution of 3,5-dibromo-1,2,4-triazole (7.125g) and triethylamine (4.81ml) in anhydrous dichloromethane (70ml) at room temperature. After 4 days, the mixture was evaporated under reduced pressure and the residue purified by column chromatography over silica, eluting with ethyl acetate/isohexane (1:4) to give the sub-title compound (7.34g) as a solid.
MS (+ve APCI) 346/348/350 ((M+H)+).

### b) 5-{[3-Bromo-1-(4-methoxyphenylmethyl)-1H-1,2,4-triazol-5-yl]thio}-3-methyl-1-(2-methylpropyl)-6-[2-(trifluoromethyl)phenylmethyl]-thieno[2,3-d]pyrimidine-2,4(1H,3H)-dione

A tetrahydrofuran solution of isopropylmagnesium chloride (2M, 0.63ml) was added to a stirred solution of the product of Preparation 1, part b) (0.50g) in anhydrous tetrahydrofuran (5ml) at 0°C under nitrogen. After 15 minutes, flowers of sulfur (0.04g) was added and stirring continued at 0°C for 30minutes. Hydrochloric acid (1M, 20ml) was added and the mixture extracted with ethyl acetate (2 x 20ml). Organic extracts were dried over anhydrous magnesium sulfate, filtered and evaporated under reduced pressure. The residue was disssolved in tetrahydrofuran (5ml) under nitrogen, saturated aqueous sodium bicarbonate solution (1ml) was added followed by sodium borohydride (0.1g) and the mixture stirred for 30 minutes. The product of step a) (0.44g) was added and stirring continued for 18 hours at room temperature then for 30 minutes at reflux. Saturated aqueous sodium bicarbonate solution (20ml) was added and the mixture extracted with ethyl acetate (2 x 20ml). Organic extracts were dried over anhydrous magnesium sulfate, filtered and evaporated under reduced pressure. The residue was purified by column chromatography over silica, eluting with ethyl acetate/isohexane (1:4) to give the sub-title compound (0.368g) as a solid.
MS (+ve APCI) 694/696 ((M+H)+).

### c) 5-[(3-Bromo-1H-1,2,4-triazol-5-yl)thio]-3-methyl-1-(2-methylpropyl)-6-[2-(trifluoromethyl)phenylmethyl]-thieno[2,3-d]pyrimidine-2,4(1H,3H)-dione

A solution of the product of step b) (0.15g) in anisole (2ml) and trifluoroacetic acid (0.5ml) was heated at reflux for 24 hours then evaporated under reduced pressure. The residue was purified by column chromatography over silica, eluting with ethyl acetate/isohexane (1:2) followed by recrystallisation from ethyl acetate/isohexane to give the title-compound (0.075g).
m.p. 174-5°C
MS (+ve APCI) 574/576 ((M+H)+)
¹H NMR (CDCl₃ ) δ 0.89 (6H, d), 2.08-2.17 (1H, m), 3.18 (3H, s), 3.71 (2H, d), 4.50 (2H, s), 7.38 (1H, d), 7.50 (1H, t), 7.62 (1H, t), 7.76 (1H, d).

### Example 28

### 3-Methyl-1-(2-methylpropyl)-5-[(5-oxo-4,5-dihydro-1H-1,2,4-triazol-3-yl)thio]-6-[2-(trifluoromethyl)phenylmethyl]-thieno[2,3-d]pyrimidine-2,4(1H,3H)-dione

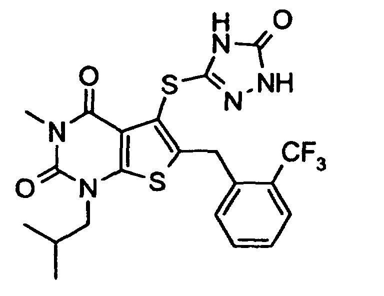

A solution of the product of Example 27, step b) (0.47g) in trifluoroacetic acid (2ml) and anisole (4ml) was heated at 90°C for 40 hours. The mixture was evaporated under reduced pressure and the residue purified by column chromatography over silica, eluting with ethyl acetate/isohexane (2:3 then 2:1) then ethyl acetate/methanol (19:1) to give the title compound (70mg) which was recrystallised from ethyl acetate/isohexane.
m.p. 205-211°C
MS (+ve APCI) 512 ((M+H)+)
¹H NMR (DMSO-D6) δ 0.87 (6H, d), 2.06-2.16 (1H, m), 3.22 (3H, s), 3.69 (1H, d), 4.47 (2H, s), 7.36 (1H, d), 7.51 (1H, t), 7.62 (1H, t), 7.77 (1H, t), 11.43 (1H, s, br), 11.46 (1H, s, br).

### Example 29

### 3-Methyl-1-(1-methylethyl)-5-(1,3-thiazol-2-ylthio)-6-[2-(trifluoromethyl)benzyl]-thieno[2,3-d]pyrimidine-2,4(1H,3H)-dione

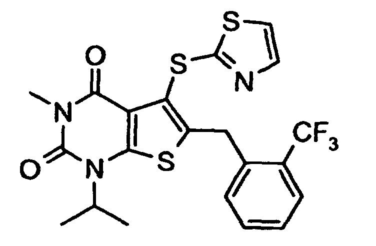

### a) Ethyl 2-(isopropylamino)-3-thiophenecarboxylate

Triethylamine (80.5ml) was added dropwise over 30 minutes to a stirred solution of 2,5-dihyhroxy-1,4-dithiane (43.9g) and ethyl cyanoacetate (61.4ml) in anhydrous dimethylformamide (200ml) at 50°C. After a further 90 minutes the mixture was cooled to room temperature, added to water (1L) and extracted with dichloromethane (4 x 50ml). Combined organic extracts were dried over anhydrous magnesium sulfate, filtered through a silica pad washing with ether (500ml) and evaporated under reduced pressure. The residual oil was redissolved in anhydrous toluene (650ml) then 2,2-dimethoxypropane (158ml) and p-toluenesulphonic acid (1g) were added. The mixture was heated under reflux under nitrogen for 5 hours then cooled to room temperature, added to saturated aqueous sodium bicarbonate solution (1L) and extracted with ether (1L). Organic extracts were dried over anhydrous sodium sulfate, filtered and evaporated under reduced pressure. The residual oil was redissolved in ethanol (250ml) and treated with sodium borohydride (8g). The mixture was stirred under nitrogen for 3 days then further sodium borohydride (8g) was added and stirring continued for 1 day. Further sodium borohydride (4g) was added and stirring continued for a further 2 days. Water (100ml) was added followed by saturated aqueous sodium bicarbonate solution (750ml) and the mixture extracted with ether (2 x 750ml). Organic extracts were dried over anhydrous magnesium sulfate, filtered and evaporated under reduced pressure. The residue was purified by column chromatography over silica, eluting with isohexane/dichloromethane (1:1) to give the subtitle compound (24.7g) as an oil.
MS (+ve APCI) 214 ((M+H)+)
¹H NMR (CDCl₃ ) δ 1.31 (6H, d), 1.33 (3H, t), 3.47-3.53 (1H, m), 4.25 (2H, q), 6.14 (1H, d), 7.02 (1H, d), 7.36 (1H, s, br).

### b) 3-Methyl-1-(1-methylethyl)-thieno[2,3-d]pyrimidine-2,4(1H,3H)-dione

Acetyl chloride (9.84ml) was added dropwise to a stirred suspension of silver cyanate (21.6g) in anhydrous toluene (100ml) under nitrogen. After 30 minutes, a solution of the product of step a) (24.6g) in anhydrous toluene (20ml) was added. The mixture was stirred for a further 2 hours then diluted with ether (500ml), filtered then washed with saturated aqueous sodium bicarbonate solution (200ml). The aqueous layer was extracted with ether (200ml) and combined organic extracts were dried over anhydrous magnesium sulfate, filtered and evaporated under reduced pressure. The residual oil was dissolved in ethanol (150ml) and treated with sodium ethoxide (23.6g). The mixture was stirred under nitrogen for 3 days then iodomethane (21.5ml) was added. The mixture was heated under reflux for 3 hours then further iodomethane (10ml) was added and heating continued for 1 hour. The mixture was cooled to room temperature, added to saturated aqueous sodium bicarbonate solution (1L) and extracted with ether (2 x 500ml). Organic extracts were dried over anhydrous magnesium sulfate, filtered and evaporated under reduced pressure. The residue was purified by column chromatography over silica, eluting with isohexane/ether (1:1) to give the sub-title compound (15.8g) as a solid.
MS (+ve APCI) 225 ((M+H)+)
¹H NMR (CDCl₃) δ 1.59 (6H, d), 1.61 (3H, t), 3.41 (3H, s), 4.64-4.80 (1H, m), 6.85 (1H, d), 7.37 (1H, d).

### c) 5-Bromo-6-{hydroxy[2-(trifluoromethyl)phenyl]methyl}-3-methyl-1-I(1-methylethyl)-thieno[2,3-d]pyrimidine-2,4(1H,3H)-dione

Bromine (1.38ml) was added to a stirred solution of the product of step b) (5.0g) in dichloromethane (100ml). After 10 minutes, the solution was evaporated under reduced pressure. The residue was dissolved in ether (200ml), washed with saturated aqueous sodium metabisulphite solution (100ml) then saturated aqueous sodium bicarbonate solution (100ml), dried over anhydrous magnesium sulfate, filtered and evaporated under reduced pressure. The residue was dissolved in anhydrous tetrahydrofuran (75ml) under nitrogen and cooled to -78°C. A solution of lithium diisopropylamide (44.6mmol) in anhydrous tetrahydrofuran (25ml) was added, the mixture warmed briefly to room temperature then re-cooled to -78°C and 2-(trifluoromethyl)benzaldehyde (5.88ml) was added. After 2 hours, saturated aqueous sodium bicarbonate solution (100ml) was added, the mixture warmed to room temperature then extracted with ether (2 x 100ml). Organic extracts were dried over anhydrous magnesium sulfate, filtered and evaporated under reduced pressure. The residue was purified by column chromatography over silica, eluting with isohexane/ether (2:1) to give the sub-title compound (6.88g) as a solid.
MS (+ve APCI) 477/479 ((M+H)+)
¹H NMR (CDCl₃ ) δ 1.59 (6H, d), 2.72 (1H, d), 3.37 (3H, s), 4.6-4.50 (1H, m), 6.58 (1H, d), 7.48 (1H, d), 7.64 (1H, t), 7.72 (1H, d), 7.79 (1H, d).

### d) 5-Bromo-3-methyl-1-(1-methylethyl)-6-[2-(trifluoromethyl)benzyl]-thieno[2,3-d]pyrimidine-2,4(1H,3H)-dione

Trifluoroacetic acid (8ml) followed by triethylsilane (4ml) were added to the product of step c) (3.8g) and the mixture stirred for 1 hour then evaporated under reduced pressure. The residue was dissolved in ethyl acetate (100ml), washed with saturated aqueous sodium bicarbonate solution (100ml), dried over anhydrous magnesium sulfate, filtered and evaporated under reduced pressure. The residue was purified by column chromatography over silica, eluting with isohexane/ethyl acetate (2:1) to give the sub-title compound (2.44g) as a solid.
MS (+ve APCI) 461/463 ((M+H)+)
¹H NMR (CDCl₃) δ 1.55 (6H, d), 3.39 (3H, s), 4.33 (2H, s), 4.30-4.45 (1H, m), 7.30 (1H, d), 7.39 (1H, t), 7.50 (1H, t), 7.70 (1H, d).

### e) 3-Methyl-1-(1-methylethyl)-5-(1,3-thiazol-2-ylthio)-6-[2-(trifluoromethyl)phenylmethyl]-thieno[2,3-d]pyrimidine-2,4(1H,3H)-dione

A solution of ethylmagnesium bromide in tetrahydrofuran (1M, 1.52ml) was added dropwise to a stirred solution of the product of step d) (0.50g) in anhydrous tetrahydrofuran (10ml) at 0°C under nitrogen. After 15 minutes, a solution of 2-(1,3-thiazol-2-yldithio)-1,3-thiazole (0.28g) in anhydrous tetrahydrofuran (5ml) was added and stirring continued at 0°C for 1 hour. Saturated aqueous sodium bicarbonate solution (20ml) was added and the mixture extracted with ethyl acetate (2 x 20ml). Organic extracts were dried over anhydrous magnesium sulfate, filtered and evaporated under reduced pressure. The residue was purified by column chromatography over silica, eluting with isohexane/ethyl acetate (4:1) and then recrystallised from ethyl acetate/isohexane to give the title-compound (0.36g).
MS (+ve ESI) 498 ((M+H)+)
¹H NMR (CDCl₃ ) δ 1.56 (6H, d), 3.33 (3H, s), 4.30-4.50 (1H, m), 4.56 (2H, s), 7.22 (1H, d), 7.27 ( 1 H, d), 7.37 (1H, t), 7.47 (3H, t), 7.66 (1H, d), 7.69 (1H, d).

### Example 30

### 3-Methyl-1-(1-methylethyl)-5-(1,3-thiazol-2-ylsulfonyl)-6-[2-(trifluoromethyl)benzyl]thieno[2,3-d]pyrimidine-2,4(1H,3H)-dione

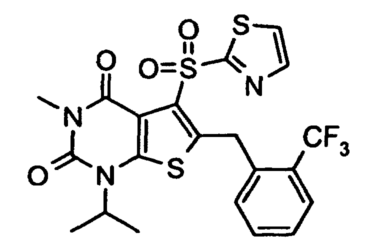

Prepared from the product of Example 17 by the method of Example 4 and purified by recrystallisation from ethyl acetate/isohexane.
MS (+ve APCI) 530 ((M+H)+)
¹H NMR (CDCl₃ ) δ 1.51 (6H, d), 3.23 (3H, s), 4.22-4.62 (1H, m), 5.03 (2H, s), 7.42-7.48 (1H, m), 7.56-7.59 (2H, m), 7.71-7.75 (2H, m), 7.91 (1H, d).

### Example 31

### 6-(9H-Fluoren-9-yl)-3-methyl-1-(2-methylpropyl)-5-(1H-1,2,4-triazol-5-ylsulfonyl)-thieno[2,3-d]pyrimidine-2,4(1H,3H)-dione

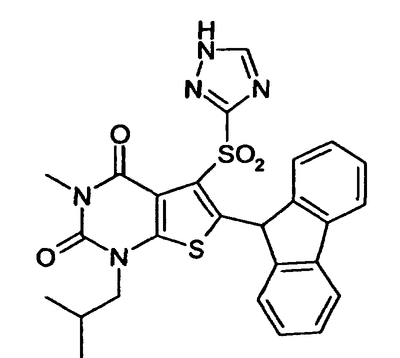

### a) 5-Bromo-6-(9-hydroxy-9H-fluoren-9-yl)-3-methyl-1-(2-methylpropyl)-thieno[2,3-d]pyrimidine-2,4(1H,3H)-dione

Prepared by the method of Preparation 1, part a), using 9-fluorenone and purified by column chromatography over silica, eluting with isohexane/ethyl acetate (3:1) to give the sub-title compound as a solid.
MS (+ve APCI) 497/9 ((M+H)+)

### b) 5-Bromo-6-(9H-fluoren-9-yl)-3-methyl-1-(2-methylpropyl)-thieno[2,3-d]pyrimidine-2,4(1H,3H)-dione

Prepared from the product of step a by the method of Preparation 1, part b) and purified by column chromatography over silica, eluting with dichloromethane/isohexane (3:1) to give the sub-title compound as a solid.
MS (+ve APCI) 481/3 ((M+H)+)

### c) Bis[1-(4-methoxyphenylmethyl)-1H-1,2,4-triazol-5-yl] disulfide

4-Methoxyphenylmethyl chloride (7.77g) was added to a stirred suspension of 1,2,4-thiadiazole-5(4H)-thione (2g) and triethylamine (6.68ml) in anhydrous tetrahydrofuran (50ml). After 16 hours, water (50ml) and ethyl acetate (50ml) were added and the mixture acidified with 2M hydrochloric acid. Undissolved solid was collected by filtration, washed with water then ethyl acetate and dried in vacuo to give the sub-title compound (2.57g).
MS (+ve APCI) 481 ((M+H)+)

### d) 6-(9H-Fluoren-9-yl)-5-{[1-(4-methoxybenzyl)-1H-1,2,4-triazol-5-yl]thio}-3-methyl-1-(2-methylpropyl)-thieno[2,3-d]pyrimidine-2,4(1H,3H)-dione

Prepared from the products of steps b) and c) by the method of Example 29, part e) and purified by column chromatography over silica, eluting with isohexane/ethyl acetate (2:1) to give the sub-title compound as a solid.
MS (+ve ESI) 642 ((M+H)+).

### e) 6-(9H-Fluoren-9-yl)-3-methyl-1-(2-methylpropyl)-5-(1H-1,2,4-triazol-5-ylsulfonyl)-thieno[2,3-d]pyrimidine-2,4(1H,3H)-dione

Aqueous hydrogen peroxide solution (30%, 0.5ml) was added to a stirred suspension of the product of step d) (0.165g) in acetic acid (1ml) and dichloromethane (1.5ml). After 6 days, sodium hydroxide solution (2M, 10ml) was added, the mixture stirred for 1 hour, diluted with water (25ml), acidified with 2M hydrochloric acid and extracted with ethyl acetate (2 x 25ml). Organic extracts were dried over anhydrous magnesium sulfate, filtered and evaporated under reduced pressure. The residue was purified by reverse-phase preparative HPLC with gradient aqueous ammonium acetate/acetonitrile elution and then further purified by column chromatography over silica, eluting with ethyl acetate/methanol (99:1) to give the title-compound (0.013g) as a solid.
MS (+ve APCI) 534 ((M+H)+)
¹H NMR (CDCl₃ ) δ 0.81 (6H, d), 1.96-2.05 (1H, m), 3.31 (3H, s), 3.54 (2H, d), 7.00 (1H, s), 7.37 (2H, t), 7.48 (2H, t), 7.76 (2H, d), 7.83 (2H, d), 8.49 (1H, s).

### Example 32

### 6-(1H-Indol-3-ylcarbonyl)-3-methyl-1-(2-methylpropyl)-5-(1H-1,2,4-triazol-3-ylthio)-thieno[2,3-d]pyrimidine-2,4(1H,3H)-dione

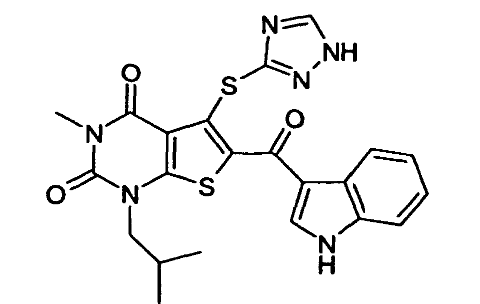

### a) 5-Bromo-6-[hydroxy[1-[[2-(trimethylsilyl)ethoxy]methyl]-1H-indol-3-yl]methyl]-3-methyl-1-(2-methylpropyl)-thieno[2,3-d]pyrimidine-2,4(1H,3H)-dione

Prepared according to the method described in Preparation 1) part a) and purified using flash silica chromatography eluting with 20% ethyl acetate in isohexane to give the subtitle compound as a yellow foam.
MS(APCI) 606/608[M+H]+

### b) 5-Bromo-3-methyl-1-(2-methylpropyl)-6-[[1-[[2-(trimethylsilyl)ethoxy]methyl]1H-indol-3-yl]carbonyl]-thieno[2,3-d]pyrimidine-2,4(1H,3H)-dione

Prepared according to the method described in Example 2) part a) and purified by flash silica chromatography eluting with a gradient of 20-100% ethyl acetate in isohexane to give the sub-title compound as a pale green solid.
MS(APCI) 590/592[M+H]+

### c) 3-Methyl-1-(2-methylpropyl)-5-(1H-1,2,4-triazol-3-ylthio)-6-[[1-[[2-(trimethylsilyl)ethoxy]methyl]-1H-indol-3-yl]carbonyl]-thieno[2,3-d]pyrimidine-2,4(1H,3H)-dione

Prepared according to the method described in Example 2) part b) and purified by flash silica chromatography eluting with 70% ethyl acetate in isohexane then 10% ethanol in dichloromethane to give the sub-title compound as a pale green solid.
MS(APCI) 611[M+H]+

### d) 6-(1H-Indol-3-ylcarbonyl)-3-methyl-1-(2-methylpropyl)-5-(1H-1,2,4-triazol-3-ylthio)-thieno[2,3-d]pyrimidine-2,4(1H,3H)-dione

The product of step c) (0.25 g,), tetra-n-butylammonium fluoride (0.39 g) and ethylenediamine (0.122 ml) were dissolved in dry dimethylformamide (6 ml) and stirred at 80°C under nitrogen for 20 hours. After cooling, the solution was partitioned between ethyl acetate and water. The organic layer was dried over anhydrous magnesium sulfate, filtered and concentrated under reduced pressure. The residue was purified by flash silica chromatography eluting with a gradient of 3-5% ethanol in dichloromethane to give the title compound as a yellow solid (0.081 g).
MS(APCI) 481[M+H]+
¹H NMR(CDCl₃) δ 1.02 (6H,d), 2.34 (1H,septet), 3.33 (3H,s), 3.83 (2H,d), 7.25-7.27 (2H,m), 7.40-7.43 (1H,m), 7.71-7.72 (1H,br.m), 8.09 (1H,m), 8.24 (1H,br.m).

### Example 33

### 6-(Hydroxy-(1H-indol-3-yl)methyl)-3-methyl-1-(2-methylpropyl)-5-(1H-1,2,4-triazol-3-ylthio)-thieno[2,3-d]pyrimidine-2,4(1H,3H)-dione

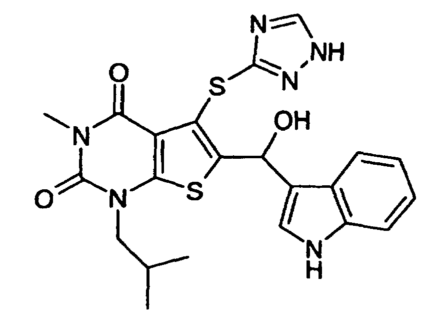

### a) 6-[Hydroxy [1-[[2-(trimethylsilyl)ethoxy]methyl]-1H-indol-3-yl]methyl]-3-methyl-1-(2-methylpropyl)-5-(1H-1,2,4-triazol-3-ylthio)-thieno[2,3-d]pyrimidine-2,4(1H,3H)-dione

Prepared using the compound of Example 32 part c) according to the method described in Example 6 and purified by flash silica chromatography eluting with 40% acetone in isohexane to give the sub-title compound as a yellow foam.
MS(APCI) 595[M-H20]+

### b) 6-(Hydroxy (1H-indol-3-yl)methyl)-3-methyl-1-(2-methylpropyl)-5-(1H-1,2,4-triazol-3-ylthio)-thieno[2,3-d]pyrimidine-2,4(1H,3H)-dione

Prepared using the product of step a) according to the method described in Example 32 part d) and purified by flash silica chromatography eluting with a gradient of 0-4% ethanol in dichloromethane to give the title compound as a brown solid.
MS(APCI) 465[M-H2O]+
¹H NMR(CDCl₃) δ 0.84-0.89 (6H,m), 2.13 (1H,septet), 3.40 (3H,s), 3.48 (1H,dd), 3.79 (1H,dd), 7.05 (2H,m), 7.09-7.10 (2H,m), 7.32-7.45 (3H,m), 8.04 (1H,s), 8.25 (1H,s).

### Example 34

### 3-Methyl-1-(2-methylpropyl)-6-(4-quinolinylcarbonyl)-5-(1,3-thiazol-2-ylthio)-thieno[2,3-d]pyrimidine-2,4(1H,3H)-dione

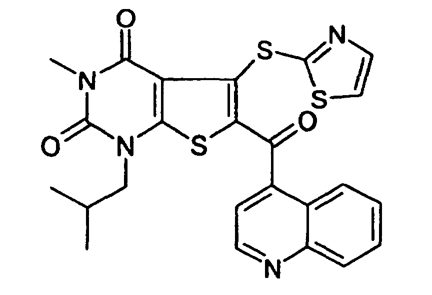

### a) 5-Bromo-6-[hydroxy(4-quinolinyl)methyl]-3-methyl-1-(2-methylpropyl)-thieno[2,3-d]pyrimidine-2,4(1H,3H)-dione

Prepared by the method of Preparation 1 part a) and purified by column chromatography over silica eluting with isohexane/ethyl acetate (3/2) to give the sub-title compound.
MS (+ve APCI) 473 ((M+H)+)
¹H NMR (CDCl₃) δ 9 (1H,d), 8.2 (1H, d), 7.9 (2H, m), 7.7 (1H, t), 7.5 (1H, t), 6.9 (1H,d), 3.4-3.8 (2H, dd), 3.4 (3H,s), 3.2 (1H,d), 2.1 (1H, m), 0.9 (3H,d), 0.9 (3H, d)

### b) 5-Bromo-3-methyl-1-(2-methylpropyl)-6-(4-quinolinylcarbonyl)-thieno[2,3-d]pyrimidine-2,4(1H,3H)-dione

Prepared from the product of step a) by the method of Example 2 part a) and purified by passage through a pad of silica eluting with dichloromethane to give the sub-title compound.
MS (+ve APCI) 473 ((M+H)+)
¹H NMR (CDCl₃) δ 9 (1H,d), 8.2 ( 1 H, d), 7.9 (2H, m), 7.6 (1H, t), 7.4 (1H, d), 3.9 (2H, d), 3.4 (3H,s), 3.2 (1H,d), 2.15 (1H, m), 1 (6H,d)

### c) 3-Methyl-1-(2-methylpropyl)-6-(4-quinolinylcarbonyl)-5-(1,3-thiazol-2-ylthio)-thieno[2,3-d]pyrimidine-2,4(1H,3H)-dione

Prepared from the product of step b) by the method of Example 2 part b) and purified by column chromatography over silica, eluting with isohexane/ethyl acetate (1/1) then isohexane/ethyl acetate (2/3) followed by recrystallisation from ethyl acetate/iso-hexane to give the title compound (0.26g).
m.p. 166.8-167.6°C
MS (+ve APCI) 509 ((M+H)+)
¹H NMR (CDCl₃) δ 1.04 (d, 6H), 2.3-2.4 (m, 1H), 3.3 (s,3H), 3.8 (d,2H), 7 (d, 1H), 7.4 (d,1H), 7.49-7.55(,2H) 7.7 (t, 1H), 7.9 (d,1H), 8.1 (d,1H), 8.8 (d,1H)

### Example 35

### 6-[Hydroxy-(4-quinolinyl)-methyl]-3-methyl-1-(2-methylpropyl)-5-(1,3-thiazol-2-ylthio)-thieno[2,3-d]pyrimidine-2,4(1H,3H)-dione

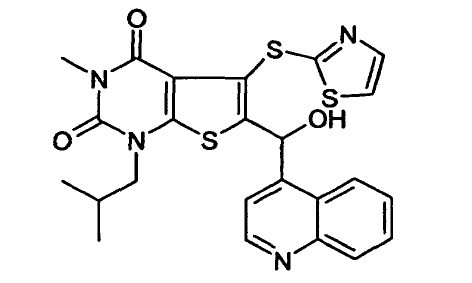

Prepared from the compound of Example 34 part c) by the method of Example 15 and purified using a reverse phase preparative HPLC with an isocratic gradient acetonitrile /0.1% ammonium aqueous solution (75/25) to give the title compound.
m.p. 182.8-184°C
MS (+ve APCI) 511 ((M+H)+)
¹H NMR (CDCl₃) δ 9.1 (1H,d), 8.2 (1H, d), 8.1 (1H, d), 7.8 (1H, d), 7.7 (2H, t), 7.5 (1H, t), 7.4 (1H, d), 7.4 (1H,d), 6.8 (1H,bs), 3.4-3.8 (2H, dd), 3.4 (3H,s), 2.1 (1H, m), 0.9 (3H,d), 0.9 (3H, d)

### Example 36

### 6-(1-Benzothien-3-ylmethyl)-3-methyl-1-(2-methylpropyl)-5-(4H-1,2,4-triazol-3-ylthio)-thieno[2,3-d]pyrimidine-2,4(1H,3H)-dione

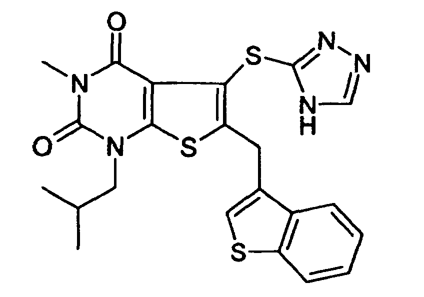

### a) 6-[1-Benzothien-3-yl (hydroxy) methyl]-5-bromo-3-methyl-1-(2-methylpropyl)-thieno[2,3-d]pyrimidine-2,4(1H,3H)-dione

Prepared by the method of Preparation 1 part a) and purified by column chromatography over silica eluting with isohexane/ethyl acetate (2.3/1) to give the sub-title compound.
MS (+ve APCI) 481 ((M+H)+)
¹H NMR (CDCl₃) δ 7.9 (1H, m), 7.7 (1H,m), 7.6 (1H, s), 7.4 (2H, m), 6.6 (1H, d), 3.5-3.9 (2H, dd), 3.4 (3H, s), 2.7 (1H, d), 2.1 (1H,m), 0.9 (3H,d), 0.9 (3H,d)

### b) 6-(1-Benzothien-3-ylcarbonyl)-5-bromo-3-methyl-1-(2-methylpropyl)-thieno[2,3-d]pyrimidine-2,4(1H,3H)-dione

Prepared from the product of step a) by the method of Example 2 part a) and purified by passage through a pad of silica eluting with dichloromethane to give the title compound.
MS (+ve APCI) 479 ((M+H)+)
¹H NMR (CDCl₃) δ 8.4 (1H, d), 8.2 (1H,s), 8.9 (1H, d), 7.5 (2H, m), 3.8 (2H, d), 3.4 (3H, s), 2.2 (1H,m), 1 (6H,d)

### c) 5-(1-Benzothien-3-yl)-5-hydroxy-7-isobutyl-9-methyl-5H-pyrimido[5',4':4,5]thieno[2,3-e][1,2,4]triazolo[3,4-b][1,3]thiazine-8,10(7H,9H)-dione

Prepared from the product of step b) by the method of Example 2 part b) and purified by column chromatography over silica, eluting with isohexane/ethyl acetate (1/1) followed by recrystallisation from ethyl acetate/iso-hexane to give the sub-title compound.
MS (+ve APCI) 498 ((M+H)+)
¹H NMR (CDCl₃) δ 8.2 (1H, s), 7.8 (1H,s), 7.8 (1H, s), 7.1 (1H, t), 7.3 (1H,t), 7 (1H,bs), 6.8 (1H,d), 3.9-3.4 (2H, dd), 3.1 (3H, s), 2.1 (1H,m), 1 (3H,d), 1 (3H,d)

### d) 6-(1-Benzothien-3-ylmethyl)-3-methyl-1-(2-methylpropyl)-5-(4H-1,2,4-triazol-3-ylthio)-thieno[2,3-d]pyrimidine-2,4(1H,3H)-dione

Prepared from the product of step c) by the method of Example 3 and was purified by chromatography over silica eluting with ethyl acetate followed by recrystallisation from dichloromethane/isohexane to give the title compound.
m.p. 237-238.2°C
MS (+ve APCI) 484 ((M+H)+)
¹H NMR (CDCl₃) δ 8 (1H, s), 7.9 (1H,m), 7.7 (1H, m), 7.35 (2H, m), 7.2 (1H,s), 4.7 (2H,s), 3.6 (2H, d), 3.4 (3H, s), 2.1 (1H,m), 0.9 (6H,d)

### Example 37

### Inhibition of PMA/ionomycin-stimulated peripheral blood mononuclear cell proliferation

The assay for PMA/ionomycin-stimulated PBMC proliferation was performed in 96-well flat bottomed microtitre plates. Compounds were prepared as 10mM stock solutions in dimethyl sulfoxide. A 50-fold dilution of this was prepared in RPMI and serial dilutions were prepared from this solution. 10µl of the 50-fold diluted stock, or dilutions of it, were added to the well to give concentrations in the assay starting at 9.5µM and going down. Into each well was placed 1 x 10⁵ PBMC, prepared from human peripheral blood from a single donor, in RPMI1640 medium supplemented with 10% human serum, 2mM glutamine and penicillin/streptomycin. Phorbol myristate acetate (PMA) (0.5ng/ml final concentration) and ionomycin (500ng/ml final concentration) were added to these cells in supplemented RPMI1640 medium (as above) so that the final volume of the assay was 0.2ml. The cells were incubated at 37°C in a humidified atmosphere at 5% carbon dioxide for 72 hours. ³H-Thymidine (0.5µCi) was added for the final 6 hours of the incubation. The level of radioactivity incorporated by the cells was then determined and this is a measure of proliferation.

The title compound of Example 1 was found to exhibit an IA₅₀ value of less than 1 x 10⁻⁶ M in the above test.

## Claims

1. A compound of general formula 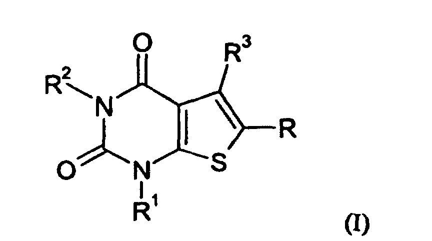 wherein:
R is -C(O)Ar¹, -C(R⁴)(R⁵)Ar¹ or Ar³;
Ar¹ represents a 5- to 10-membered aromatic ring system wherein up to 3 ring atoms may be heteroatoms independently selected from nitrogen, oxygen and sulphur, the ring system being optionally substituted by one or more substituents independently selected from C₁₋₄ alkyl, C₁₋₄ alkoxy, halogen, trifluoromethyl, oxo, nitro, cyano, NR⁶R⁷ and -CH₂NR⁸R⁹;
R¹ and R² each independently represent a hydrogen atom, C₁₋₆ alkyl, C₃₋₆ alkenyl, CH₂C₃₋₅ cycloalkyl or C₃₋₆ cycloalkyl;
R³ represents a group X-Ar²;
X represents a group S(O)ₙ, C(O) or CH(OH);
n is 0, 1 or 2;
Ar² represents a pyrrolyl, thiazolyl, thiadiazolyl, oxazolyl, imidazolyl or triazolyl ring, the ring being optionally substituted by one or more substituents independently selected from C₁₋₄ alkyl, C₁₋₄ alkoxy, C₁₋₄ alkylthio, halogen, trifluoromethyl, oxo, hydroxyl, amino, nitro, cyano and benzyl;
R⁴ represents a hydrogen atom or C₁₋₄ alkyl;
R⁵ represents a hydrogen atom or hydroxyl group;
R⁶ and R⁷ each independently represent a hydrogen atom or C₁₋₄ alkyl, or together with the nitrogen atom to which they are attached form a 5- to 7-membered saturated heterocyclic ring;
R⁸ and R⁹ each independently represent a hydrogen atom or C₁₋₄ alkyl, or together with the nitrogen atom to which they are attached form a 5- to 7-membered saturated heterocyclic ring; and
Ar³ represents acenaphthenyl, indanyl or fluorenyl, each of which may be optionally substituted by one or more substituents independently selected from C₁₋₄ alkyl, C₁₋₄ alkoxy, halogen or trifluoromethyl;
or a pharmaceutically acceptable salt or solvate thereof.

2. A compound according to claim 1, wherein R represents -C(R⁴)(R⁵)Ar¹.

3. A compound according to claim 1 or claim 2, wherein Ar¹ represents phenyl, naphthyl, pyrazolyl, thienyl, oxazolyl, imidazolyl, pyridinyl, pyridopyrrolyl, benzimidazolyl, indazolyl, benzothiazolyl, benzoxazolyl, thiazolyl or benzotriazolyl, each of which may be optionally substituted by one to four substituents independently selected from C₁₋₄ alkyl, C₁₋₄ alkoxy, halogen, trifluoromethyl, oxo, nitro, cyano, NR⁶R⁷ and -CH₂NR⁸R⁹.

4. A compound according to any one of claims 1 to 3, wherein R¹ and R² each independently represent a C₁₋₄ alkyl group.

5. A compound according to any one of claims 1 to 4, wherein Ar² represents thiazolyl or triazolyl, each of which may be optionally substituted by one to three substituents independently selected from C₁₋₄ alkyl..

6. A compound of formula (I), or a pharmaceutically acceptable salt or solvate thereof, according to claim 1 being:
1-Isobutyl-3-methyl-5-[(1-methyl-1H-pyrrol-2-yl)carbonyl]-6-[2-(trifluoromethyl)benzyl]-thieno[2,3-d]pyrimidine-2,4(1H,3H)-dione,
3-Methyl-1-(2-methylpropyl)-5-(1H-1,2,4-triazol-3-ylthio)-6-[2-(trifluoromethyl)benzoyl]-thieno[2,3-d]pyrimidine-2,4(1H,3H)-dione,
3-Methyl-1-(2-methylpropyl)-5-(1H-1,2,4-triazol-3-ylthio)-6-[[2-(trifluoromethyl)phenyl]methyl]-thieno[2,3-d]pyrimidine-2,4(1H,3H)-dione,
3-Methyl-1-(2-methylpropyl)-5-(1H-1,2,4-triazol-3-ylsulfonyl)-6-[[2-(trifluoromethyl)phenyl]methyl]-thieno[2,3-d]pyrimidine-2,4(1H,3H)-dione,
3-Methyl-1-(2-methylpropyl)-5-(2-thiazolylthio)-6-[2-(trifluoromethyl)benzoyl]-thieno[2,3-d]pyrimidine-2,4(1H,3H)-dione,
3-Methyl-1-(2-methylpropyl)-5-[[5-(methylthio)-1,3,4-thiadiazol-2-yl]thio]-6-[2-(trifluoromethyl)benzoyl]-thieno[2,3-d]pyrimidine-2,4(1H,3H)-dione,
3-Methyl-5-[(4-methyl-2-oxazolyl)thio]-1-(2-methylpropyl)-6-[2-(trifluoromethyl)benzoyl]-thieno[2,3-d]pyrimidine-2,4(1H,3H)-dione,
3-Methyl-1-(2-methylpropyl)-5-[(5-methyl-1H-1,2,4-triazol-3-yl)thio]-6-[2-(trifluoromethyl)benzoyl]-thieno[2,3-d]pyrimidine-2,4(1H,3H)-dione,
3-Methyl-1-(2-methylpropyl)-5-[(5-hydroxy-4-(1-methylethyl)-1H-1,2,4-triazol-3-yl)thio]-6-[2-(trifluoromethyl)benzoyl]-thieno[2,3-d]pyrimidine-2,4(1H,3H)-dione,
3-Methyl-1-(2-methylpropyl)-5-[(5-methyl-1,3,4-thiadiazol-2-yl)thio]-6-[2-(trifluoromethyl)benzoyl]-thieno[2,3-d]pyrimidine-2,4(1H,3H)-dione,
5-(1H-Imidazol-2-ylthio)-3-methyl-1-(2-methylpropyl)-6-[2-(trifluoromethyl)benzoyl]-thieno[2,3-d]pyrimidine-2,4(1H,3H)-dione,
5-[[4-(1,1-Dimethylethyl)-4H-1,2,4-triazol-3-yl]thio]-3-methyl-1-(2-methylpropyl)-6-[2-(trifluoromethyl)benzoyl]-thieno[2,3-d]pyrimidine-2,4(1H,3H)-dione,
5-[(5-Amino-1,3,4-thiadiazol-2-yl)thio]-3-methyl-1-(2-methylpropyl)-6-[2-(trifluoromethyl)benzoyl]-thieno[2,3-d]pyrimidine-2,4(1H,3H)-dione,
3-Methyl-1-(2-methylpropyl)-5-(1,3,4-thiadiazol-2-ylthio)-6-[2-(trifluoromethyl)benzoyl]-thieno[2,3-d]pyrimidine-2,4(1H,3H)-dione,
6-[Hydroxy[2-(trifluoromethyl)phenyl]methyl]-5-(1H-imidazol-2-ylthio)-3-methyl-1-(2-methylpropyl)-thieno[2,3-d]pyrimidine-2,4(1H,3H)-dione,
6-[Hydroxy[2-(trifluoromethyl)phenyl]methyl]-3-methyl-1-(2-methylpropyl)-5-(1H-1,2,4-triazol-3-ylthio)-thieno[2,3-d]pyrimidine-2,4(1H,3H)-dione,
3-Methyl-1-(2-methylpropyl)-5-(2-thiazolylthio)-6-[[2-(trifluoromethyl)-phenyl]methyl]-thieno[2,3-d]pyrimidine-2,4(1H,3H)-dione,
3-Methyl-1-(2-methylpropyl)-5-[(5-methyl-1H-1,2,4-triazol-3-yl)thio]-6-[[2-(trifluoromethyl)phenyl]methyl]-thieno[2,3-d]pyrimidine-2,4(1H,3H)-dione dihydrochloride,
5-( 1H-Imidazol-2-ylthio)-3-methyl-1-(2-methylpropyl)-6-[[2-(trifluoromethyl)phenyl]methyl]-thieno[2,3-d]pyrimidine-2,4(1H,3H)-dione,
3-Methyl-1-(2-methylpropyl)-5-(1,3,4-thiadiazol-2-ylthio)-6-[2-(trifluoromethyl)benzyl]-thieno[2,3-d]pyrimidine-2,4(1H,3H)-dione,
5-[(5-Amino-1,3,4-thiadiazol-2-yl)thio]-3-methyl-1-(2-methylpropyl)-6-[2-(trifluoromethyl)benzyl]-thieno[2,3-d]pyrimidine-2,4(1H,3H)-dione,
3-Methyl-1-(2-methylpropyl)-5-[(5-methyl-1H-1,2,4-triazol-3-yl)sulfonyl]-6-[[2-(trifluoromethyl)phenyl]methyl]-thieno[2,3-d]pyrimidine-2,4(1H,3H)-dione,
5-(1H-Imidazol-2-ylsulfonyl)-3-methyl-1-(2-methylpropyl)-6-[[2-(trifluoromethyl)phenyl]methyl]-thieno[2,3-d]pyrimidine-2,4(1H,3H)-dione,
6-[Hydroxy[2-(trifluoromethyl)phenyl]methyl]-3-methyl-1-(2-methylpropyl)-5-(1H-1,2,4-triazol-3-ylsulfonyl)-thieno[2,3-d]pyrimidine-2,4(1H,3H)-dione,
3-Methyl-1-(2-methylpropyl)-5-(1,3-thiazol-2-ylsulfonyl)-6-[2-(trifluoromethyl)benzyl]-thieno[2,3-d]pyrimidine-2,4(1H,3H)-dione,
3-Methyl-1-(2-methylpropyl)-5-(1,3,4-thiadiazol-2-ylsulfonyl)-6-[2-(trifluoromethyl)benzyl]-thieno[2,3-d]pyrimidine-2,4(1H,3H)-dione,
5-[(3-Bromo-1H-1,2,4-triazol-5-yl)thio]-3-methyl-1-(2-methylpropyl)-6-[2-(trifluoromethyl)phenylmethyl]-thieno[2,3-d]pyrimidine-2,4(1H,3H)-dione,
3-Methyl-1-(2-methylpropyl)-5-[(5-oxo-4,5-dihydro-1H-1,2,4-triazol-3-yl)thio]-6-[2-(trifluoromethyl)phenylmethyl]-thieno[2,3-d]pyrimidine-2,4(1H,3H)-dione,
3-Methyl-1-(1-methylethyl)-5-(1,3-thiazol-2-ylthio)-6-[2-(trifluoromethyl)benzyl]-thieno[2,3-d]pyrimidine-2,4(1H,3H)-dione,
3-Methyl-1-(1-methylethyl)-5-(1,3-thiazol-2-ylsulfonyl)-6-[2-(trifluoromethyl)benzyl]thieno[2,3-d]pyrimidine-2,4(1H,3H)-dione,
6-(9H-Fluoren-9-yl)-3-methyl-1-(2-methylpropyl)-5-(1H-1,2,4-triazol-5-ylsulfonyl)-thieno[2,3-d]pyrimidine-2,4(1H,3H)-dione,
6-( 1H-Indol-3-ylcarbonyl)-3-methyl-1-(2-methylpropyl)-5-(1H-1,2,4-triazol-3-ylthio)-thieno[2,3-d]pyrimidine-2,4(1H,3H)-dione,
6-(Hydroxy-(1H-indol-3-yl)methyl)-3-methyl-1-(2-methylpropyl)-5-(1H-1,2,4-triazol-3-ylthio)-thieno[2,3-d]pyrimidine-2,4(1H,3H)-dione,
3-Methyl-1-(2-methylpropyl)-6-(4-quinolinylcarbonyl)-5-(1,3-thiazol-2-ylthio)-thieno[2,3-d]pyrimidine-2,4(1 H,3H)-dione,
6-[Hydroxy-(4-quinolinyl)-methyl]-3-methyl-1-(2-methylpropyl)-5-(1,3-thiazol-2-ylthio)-thieno[2,3-d]pyrimidine-2,4(1H,3H)-dione,
6-( 1-Benzothien-3-ylmethyl)-3-methyl-1-(2-methylpropyl)-5-(4H-1,2,4-triazol-3-ylthio)-thieno[2,3-d]pyrimidine-2,4(1H,3H)-dione,
or a pharmaceutically acceptable salt or solvate thereof.

7. A process for the preparation of a compound of formula (I) as defined in claim I which comprises,
(a) when R represents C(O)Ar¹ and X represents a sulphur atom, reacting a compound of general formula 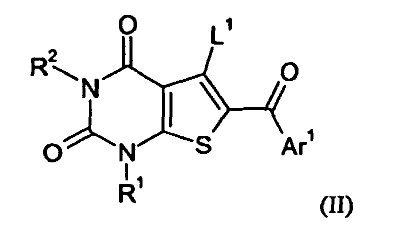 wherein L¹ represents a leaving group and R¹, R² and Ar¹ are as defined in formula (I), with a compound of general formula (III), HS-Ar², wherein Ar² is as defined in formula (I), in the presence of a base; or
(b) when R represents -C(R⁴)(R⁵)Ar¹, R⁴ represents a hydrogen atom, R⁵ represents hydroxyl and X represents a sulphur atom, reacting a corresponding compound of formula (I) in which R represents C(O)Ar¹ with a suitable reducing agent; or
(c) when R represents -C(R⁴)(R⁵)Ar¹, R⁴ represents a hydrogen atom, R⁵ represents a hydrogen atom and X represents a sulphur atom, reacting a corresponding compound of formula (I) in which R represents C(O)Ar¹ with a suitable reducing agent, in the presence of a Lewis acid; or
(d) when R represents -C(R⁴)(R⁵)Ar¹ and R⁴ represents C₁₋₄ alkyl, reacting a corresponding compound of formula (I) in which R represents C(O)Ar¹ with a suitable Grignard reagent, followed by reaction with a dehydrating agent in the presence of a base, and then followed by a hydrogenation reaction; or
(e) when X represents SO or SO₂, reacting a corresponding compound of formula (I) in which X is a sulphur atom with a suitable oxidising agent; or
(f) when X represents CH(OH), reacting a compound of general formula 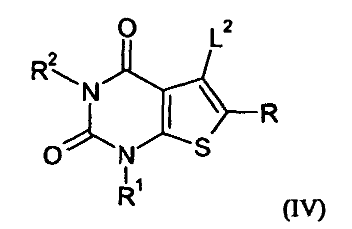 wherein L² represents a leaving group and R, R¹ and R² are as defined in formula (I), with a suitable Grignard reagent and then with a compound of general formula (V), Ar²CHO, wherein Ar² is as defined in formula (I); or
(g) when X represents C(O), reacting a corresponding compound of formula (I) in which X represents CH(OH) with a suitable oxidising agent; or
(h) when X represents a sulphur atom, reacting a compound of formula (IV) as defined in (f) above with a suitable alkyl lithium compound or Grignard reagent and then with a compound of general formula (VI), Ar²-S-S-Ar², wherein Ar² is as defined in formula (I);
and optionally after (a), (b), (c), (d), (e), (f), (g) or (h) forming a pharmaceutically acceptable salt or solvate of the compound of formula (I) obtained.

8. A pharmaceutical composition comprising a compound of formula (I) or a pharmaceutically acceptable salt or solvate thereof according to any one of claims 1 to 6 in association with a pharmaceutically acceptable adjuvant, diluent or carrier.

9. A process for the preparation of a pharmaceutical composition as claimed in claim 8 which comprises mixing a compound of formula (I) or a pharmaceutically acceptable salt or solvate thereof according to any one of claims 1 to 6 with a pharmaceutically acceptable adjuvant, diluent or carrier.

10. A compound of formula (I) or a pharmaceutically acceptable salt or solvate thereof as claimed in any one of claims 1 to 6 for use in therapy.

11. Use of a compound of formula (I) or a pharmaceutically acceptable salt or solvate thereof as claimed in any one of claims 1 to 6 in the manufacture of a medicament for use in therapy.

12. Use of a compound of formula (I) or a pharmaceutically acceptable salt or solvate thereof as claimed in any one of claims 1 to 6 in the manufacture of a medicament for use in the treatment of an obstructive airways disease.

13. Use according to claim 12, wherein the obstructive airways disease is asthma or chronic obstructive pulmonary disease.

14. Use of a compound of formula (I) or a pharmaceutically acceptable salt or solvate thereof as claimed in any one of claims 1 to 6 in the manufacture of a medicament for use in the treatment of allograft rejection.

## Patentansprüche

1. Verbindung der allgemeinen Formel: 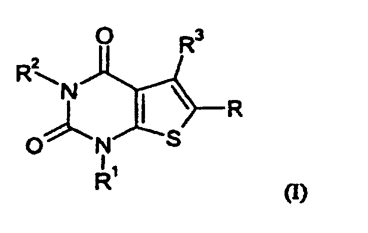 worin:
R für -C(O)Ar¹, -C(R⁴) (R⁵)Ar¹ oder Ar³ steht;
Ar¹ für ein 5- bis 10-gliedriges aromatisches Ringsystem steht, wobei es sich bei bis zu 3 Ringatomen um unabhängig voneinander unter Stickstoff, Sauerstoff und Schwefel ausgewählte Heteroatome handeln kann und das Ringsystem gegebenenfalls durch einen oder mehrere unabhängig voneinander unter C₁₋₄-Alkyl, C₁₋₄-Alkoxy, Halogen, Trifluormethyl, Oxo, Nitro, Cyano, NR⁶R⁷ und -CH₂NR⁸R⁹ ausgewählte Substituenten substituiert ist;
R¹ und R² jeweils unabhängig voneinander für ein Wasserstoffatom, C₁₋₆-Alkyl, C₃₋₆-Alkenyl, CH₂-C₃₋₅-Cycloalkyl oder C₃₋₆-Cycloalkyl stehen;
R³ für eine X-Ar²-Gruppe steht;
X für eine S(O)ₙ-, C(O)-, oder CH(OH)-Gruppe steht;
n für 0, 1 oder 2 steht;
Ar² für einen Pyrrolyl-, Thiazolyl-, Thiadiazolyl-, Oxazolyl-, Imidazolyl- oder Triazolylring steht, der gegebenenfalls durch einen oder mehrere unabhängig voneinander unter C₁₋₄-Alkyl, C₁₋₄-Alkoxy, C₁₋₄-Alkylthio, Halogen, Trifluormethyl, Oxo, Hydroxyl, Amino, Nitro, Cyano und Benzyl ausgewählte Substituenten substituiert ist;
R⁴ für ein Wasserstoffatom oder C₁₋₄-Alkyl steht;
R⁵ für ein Wasserstoffatom oder eine Hydroxylgruppe steht;
R⁶ und R⁷ jeweils unabhängig voneinander für ein Wasserstoffatom oder C₁₋₄-Alkyl stehen oder gemeinsam mit dem Stickstoffatom, an das sie gebunden sind, einen 5- bis 7-gliedrigen gesättigten heterocyclischen Ring bilden;
R⁸ und R⁹ jeweils unabhängig voneinander für ein Wasserstoffatom oder C₁₋₄-Alkyl stehen oder gemeinsam mit dem Stickstoffatom, an das sie gebunden sind, einen 5- bis 7-gliedrigen gesättigten heterocyclischen Ring bilden; und
Ar³ für Acenaphthyl, Indanyl oder Fluorenyl steht, wobei diese Gruppen jeweils durch einen oder mehrere unabhängig voneinander unter C₁₋₄-Alkyl, C₁₋₄-Alkoxy, Halogen oder Trifluormethyl ausgewählte Substituenten substituiert sein können;
oder ein pharmazeutisch unbedenkliches Salz oder Solvat davon.

2. Verbindung nach Anspruch 1, worin R für -C(R⁴)(R⁵)Ar¹ steht.

3. Verbindung nach Anspruch 1 oder 2, worin Ar¹ für Phenyl, Naphthyl, Pyrazolyl, Thienyl, Oxazolyl, Imidazolyl, Pyridinyl, Pyridopyrrolyl, Benzimidazolyl, Indazolyl, Benzothiazolyl, Benzoxazolyl, Thiazolyl oder Benzotriazolyl steht, wobei diese Gruppen jeweils gegebenenfalls durch einen bis vier unabhängig voneinander unter C₁₋₄-Alkyl, C₁₋₄-Alkoxy, Halogen, Trifluormethyl, Oxo, Nitro, Cyano, NR⁶R⁷ und -CH₂NR⁸R⁹ ausgewählte Substituenten substituiert sein können.

4. Verbindung nach einem der Ansprüche 1 bis 3, worin R¹ und R² jeweils unabhängig voneinander für eine C₁₋₄-Alkylgruppe stehen.

5. Verbindung nach einem der Ansprüche 1 bis 4, worin Ar² für Thiazolyl oder Triazolyl steht, wobei diese Gruppen gegebenenfalls durch einen bis drei unabhängig voneinander unter C₁₋₄-Alkyl ausgewählte Substituenten substituiert sein können.

6. Verbindung der Formel (I) oder ein pharmazeutisch unbedenkliches Salz oder Solvat davon nach Anspruch 1, wobei es sich um:
1-Isobutyl-3-methyl-5-[(1-methyl-1H-pyrrol-2-yl)-carbonyl]-6-[2-(trifluormethyl)benzyl]thieno[2,3-d]pyrimidin-2,4(1H,3H)-dion,
3-Methyl-1-(2-methylpropyl)-5-(1H-1,2,4-triazol-3-ylthio)-6-[2-(trifluormethyl)benzoyl]thieno[2,3-d]pyrimidin-2,4(1H,3H)-dion,
3-Methyl-1-(2-methylpropyl)-5-(1H-1,2,4-triazol-3-ylthio)-6-[[2-(trifluormethyl)phenyl]methyl]-thieno[2,3-d]pyrimidin-2,4(1H,3H)-dion,
3-Methyl-1-(2-methylpropyl)-5-(1H-1,2,4-triazol-3-ylsulfonyl)-6-[[2-(trifluormethyl)phenyl]methyl]-thieno[2,3-d]pyrimidin-2,4(1H,3H)-dion,
3-Methyl-1-(2-methylpropyl)-5-(2-thiazolylthio)-6-[2-(trifluormethyl)benzoyl]thieno[2,3-d]pyrimidin-2,4(1H,3H)-dion,
3-Methyl-1-(2-methylpropyl)-5-[[5-(methylthio)-1,3,4-thiadiazol-2-yl]thio]-6-[2-(trifluormethyl)-benzoyl]thieno[2,3-d]pyrimidin-2,4(1H,3H)-dion,
3-Methyl-5-[(4-methyl-2-oxazolyl)thio]-1-(2-methylpropyl)-6-[2-(trifluormethyl)benzoyl]thieno-[2,3-d]pyrimidin-2,4(1H,3H)-dion,
3-Methyl-1-(2-methylpropyl)-5-[(5-methyl-1H-1,2,4-triazol-3-yl)thio]-6-[2-(trifluormethyl)-benzoyl]thieno[2,3-d]pyrimidin-2,4(1H,3H)-dion,
3-Methyl-1-(2-methylpropyl)-5-[(5-hydroxy-4-(1-methylethyl)-1H-1,2,4-triazol-3-yl)thio]-6-[2-(trifluormethyl)benzoyl]thieno[2,3-d]pyrimidin-2,4(1H,3H)-dion,
3-Methyl-1-(2-methylpropyl)-5-[(5-methyl-1,3,4-thiadiazol-2-yl)thio]-6-[2-(trifluormethyl)-benzoyl]thieno[2,3-d]pyrimidin-2,4(1H,3H)-dion,
5-(1H-Imidazol-2-ylthio)-3-methyl-1-(2-methylpropyl)-6-[2-(trifluormethyl)benzoyl]thieno[2,3-d]pyrimidin-2,4(1H,3H)-dion,
5-[[4-(1,1-Dimethylethyl)-4H-1,2,4-triazol-3-yl]thio]-3-methyl-1-(2-methylpropyl)-6-[2-(trifluormethyl)benzoyl]thieno[2,3-d]pyrimidin-2,4(1H,3H)-dion,
5-[(5-Amino-1,3,4-thiadiazol-2-yl)thio]-3-methyl-1-(2-methylpropyl)-6-[2-(trifluormethyl)benzoyl]-thieno[2,3-d]pyrimidin-2,4(1H,3H)-dion,
3-Methyl-1-(2-methylpropyl)-5-(1,3,4-thiadiazol-2-ylthio)-6-[2-(trifluormethyl)benzoyl]thieno[2,3-d]pyrimidin-2,4(1H,3H)-dion,
6-[Hydroxy-[2-(trifluormethyl)phenyl]methyl]-5-(1H-imidazol-2-ylthio)-3-methyl-1-(2-methylpropyl)thieno[2,3-d]pyrimidin-2,4(1H,3H)-dion,
6-[Hydroxy-[2-(trifluormethyl)phenyl]methyl]-3-methyl-1-(2-methylpropyl)-5-(1H-1,2,4-triazol-3-ylthio)thieno[2,3-d]pyrimidin-2,4(1H,3H)-dion,
3-Methyl-1-(2-methylpropyl)-5-(2-thiazolylthio)-6-[[2-(trifluormethyl)phenyl]methyl]thieno[2,3-d]-pyrimidin-2,4(1H,3H)-dion,
3-Methyl-1-(2-methylpropyl)-5-[(5-methyl-1H-1,2,4-triazol-3-yl)thio]-6-[[2-(trifluormethyl)phenyl]-methyl]thieno[2,3-d]pyrimidin-2,4(1H,3H)-dion-dihydrochlorid,
5-(1H-Imidazol-2-ylthio)-3-methyl-1-(2-methylpropyl)-6-[[2-(trifluormethyl)phenyl]methyl]-thieno[2,3-d]pyrimidin-2,4(1H,3H)-dion,
3-Methyl-1-(2-methylpropyl)-5-(1,3,4-thiadiazol-2-ylthio)-6-[2-(trifluormethyl)benzyl]thieno[2,3-d]-pyrimidin-2,4(1H,3H)-dion,
5-[(5-Amino-1,3,4-thiadiazol-2-yl)thio]-3-methyl-1-(2-methylpropyl)-6-[2-(trifluormethyl)benzyl]-thieno[2,3-d]pyrimidin-2,4(1H,3H)-dion,
3-Methyl-1-(2-methylpropyl)-5-[5-methyl-1H-1,2,4-triazol-3-yl)sulfonyl]-6-[[2-(trifluormethyl)-phenyl]methyl]thieno[2,3-d]pyrimidin-2,4(1H,3H)-dion,
5-(1H-Imidazol-2-ylsulfonyl)-3-methyl-1-(2-methylpropyl)-6-[[2-(trifluormethyl)phenyl]methyl]-thieno[2,3-d]pyrimidin-2,4(1H,3H)-dion,
6-[Hydroxy-[2-(trifluormethyl)phenyl]methyl]-3-methyl-1-(2-methylpropyl)-5-(1H-1,2,4-triazol-3-ylsulfonyl)thieno[2,3-d]pyrimidin-2,4(1H,3H)-dion,
3-Methyl-1-(2-methylpropyl)-5-(1,3-thiazol-2-ylsulfonyl)-6-[2-(trifluormethyl)benzyl]thieno[2,3-d]pyrimidin-2,4(1H,3H)-dion,
3-Methyl-1-(2-methylpropyl)-5-(1,3,4-thiadiazol-2-ylsulfonyl)-6-[2-(trifluormethyl)benzyl]thieno-[2,3-d]pyrimidin-2,4(1H,3H)-dion,
5-[(3-Brom-1H-1,2,4-triazol-5-yl)thio]-3-methyl-1-(2-methylpropyl)-6-[2-(trifluormethyl)phenylmethyl]thieno[2,3-d]pyrimidin-2,4(1H,3H)-dion,
3-Methyl-1-(2-methylpropyl)-5-[(5-oxo-4,5-dihydro-1H-1,2,4-triazol-3-yl)thio]-6-[2-(trifluormethyl)-phenylmethyl]thieno[2,3-d]pyrimidin-2,4(1H,3H)-dion,
3-Methyl-1-(1-methylethyl)-5-(1,3-thiazol-2-ylthio)-6-[2-(trifluormethyl)benzyl]thieno[2,3-d]-pyrimidin-2,4(1H,3H)-dion,
3-Methyl-1-(1-methylethyl)-5-(1,3-thiazol-2-ylsulfonyl)-6-[2-(trifluormethyl)benzyl]thieno[2,3-d]pyrimidin-2,4(1H,3H)-dion,
6-(9H-Fluoren-9-yl)-3-methyl-1-(2-methylpropyl)-5-(1H-1,2,4-triazol-5-ylsulfonyl)thieno[2,3-d]-pyrimidin-2,4(1H,3H)-dion,
6-(1H-Indol-3-ylcarbonyl)-3-methyl-1-(2-methylpropyl)-5-(1H-1,2,4-triazol-3-ylthio)thieno[2,3-d]pyrimidin-2,4(1H,3H)-dion,
6-(Hydroxy-(1H-indol-3-yl)methyl)-3-methyl-1-(2-methylpropyl)-5-(1H-1,2,4-triazol-3-ylthio)thieno-[2,3-d]pyrimidin-2,4(1H,3H)-dion,
3-Methyl-1-(2-methylpropyl)-6-(4-chinolinylcarbonyl)-5-(1,3-thiazol-2-ylthio)thieno[2,3-d]-pyrimidin-2,4(1H,3H)-dion,
6-[Hydroxy-(4-chinolinyl)methyl)-3-methyl-1-(2-methylpropyl)-5-(1,3-thiazol-2-ylthio)thieno[2,3-d]pyrimidin-2,4(1H,3H)-dion,
6-(1-Benzothien-3-ylmethyl)-3-methyl-1-(2-methylpropyl)-5-(4H-1,2,4-triazol-3-ylthio)thieno[2,3-d]pyrimidin-2,4(1H,3H)-dion,
oder ein pharmazeutisch unbedenkliches Salz oder Solvat davon handelt.

7. Verfahren zur Herstellung einer Verbindung der Formel (I) nach Anspruch 1, bei dem man:
(a) wenn R für C(O)Ar¹ steht und X für ein Schwefelatom steht, eine Verbindung der allgemeinen Formel 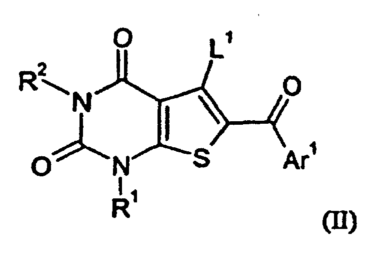 worin L¹ für eine Abgangsgruppe steht und R¹, R² und Ar¹ die unter Formel (I) angegebene Bedeutung besitzen, in Gegenwart einer Base mit einer Verbindung der allgemeinen Formel (III), HS-Ar², worin Ar² die unter Formel (I) angegebene Bedeutung besitzt, umsetzt; oder
(b) wenn R für -C(R⁴)(R⁵)Ar¹ steht, R⁴ für ein Wasserstoffatom steht, R⁵ für eine Hydroxylgruppe steht und X für ein Schwefelatom steht, eine entsprechende Verbindung der Formel (I), worin R für C(O)Ar¹ steht, mit einem geeigneten Reduktionsmittel umsetzt; oder
(c) wenn R für -C(R⁴) (R⁵)Ar¹ steht, R⁴ für ein Wasserstoffatom steht, R⁵ für ein Wasserstoffatom steht und X für ein Schwefelatom steht, eine entsprechende Verbindung der Formel (I), worin R für C(O)Ar¹ steht, in Gegenwart einer Lewis-Säure mit einem geeigneten Reduktionsmittel umsetzt; oder
(d) wenn R für -C(R⁴) (R⁵)Ar¹ steht und R⁴ für C₁₋₄-Alkyl steht, eine entsprechende Verbindung der Formel (I), worin R für C(O)Ar¹ steht, mit einem geeigneten Grignard-Reagenz und danach in Gegenwart einer Base mit einem Dehydratisierungsmittel umsetzt und danach eine Hydrierungsreaktion durchführt; oder
(e) wenn X für SO oder SO₂ steht, eine entsprechende Verbindung der Formel (I), worin X für ein Schwefelatom steht, mit einem geeigneten Oxidationsmittel umsetzt; oder
(f) wenn X für CH(OH) steht, eine Verbindung der allgemeinen Formel 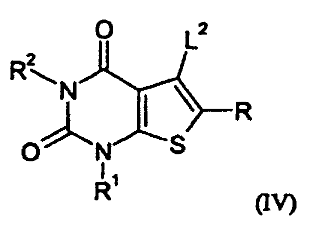 worin L² für eine Abgangsgruppe steht und R, R¹ und R² die unter Formel (I) angegebene Bedeutung besitzen, mit einem geeigneten Grignard-Reagenz und danach mit einer Verbindung der allgemeinen Formel (V), Ar²CHO, worin Ar² die unter Formel (I) angegebene Bedeutung besitzt, umsetzt; oder
(g) wenn X für C(O) steht, eine entsprechende Verbindung der Formel (I), worin X für CH(OH) steht, mit einem geeigneten Oxidationsmittel umsetzt; oder
(h) wenn X für ein Schwefelatom steht, eine Verbindung der Formel (IV) gemäß obiger Definition unter (f) mit einer geeigneten Alkyllithiumverbindung oder einem geeigneten Grignard-Reagenz und danach mit einer Verbindung der allgemeinen Formel (VI), Ar²-S-S-Ar², worin Ar² die unter Formel (I) angegebene Bedeutung besitzt, umsetzt;
und gegebenenfalls nach (a), (b), (c), (d), (e), (f), (g) oder (h) ein pharmazeutisch unbedenkliches Salz oder Solvat der Verbindung der Formel (I) bildet.

8. Pharmazeutische Zusammensetzung, enthaltend eine Verbindung der Formel (I) oder ein pharmazeutisch unbedenkliches Salz oder Solvat davon nach einem der Ansprüche 1 bis 6 zusammen mit einem pharmazeutisch unbedenklichen Hilfsstoff, Verdünnungsmittel oder Träger.

9. Verfahren zur Herstellung einer pharmazeutischen Zusammensetzung nach Anspruch 8, bei dem man eine Verbindung der Formel (I) oder ein pharmazeutisch unbedenkliches Salz oder Solvat davon nach einem der Ansprüche 1 bis 6 mit einem pharmazeutisch unbedenklichen Hilfsstoff, Verdünnungsmittel oder Träger vermischt.

10. Verbindung der Formel (I) oder ein pharmazeutisch unbedenkliches Salz oder Solvat davon nach einem der Ansprüche 1 bis 6 zur Verwendung bei der Therapie.

11. Verwendung einer Verbindung der Formel (I) oder eines pharmazeutisch unbedenklichen Salzes oder Solvats davon nach einem der Ansprüche 1 bis 6 zur Herstellung eines Arzneimittels zur Verwendung bei der Therapie.

12. Verwendung einer Verbindung der Formel (I) oder eines pharmazeutisch unbedenklichen Salzes oder Solvats davon nach einem der Ansprüche 1 bis 6 zur Herstellung eines Arzneimittels zur Verwendung bei der Behandlung einer obstruktiven Atemwegserkrankung.

13. Verwendung nach Anspruch 12, bei der es sich bei der obstruktiven Atemwegserkrankung um Asthma oder chronische obstruktive Lungenerkrankung handelt.

14. Verwendung einer Verbindung der Formel (I) oder eines pharmazeutisch unbedenklichen Salzes oder Solvats davon nach einem der Ansprüche 1 bis 6 zur Herstellung eines Arzneimittels zur Verwendung bei der Behandlung von Allotransplantat-Abstoßung.

## Revendications

1. Composé de formule générale 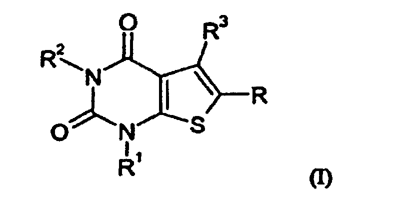 dans laquelle
R est -C(O)Ar¹, -C(R⁴) (R⁵)Ar¹ ou Ar³ ;
dans laquelle Ar¹ représente un système de cycle aromatique à 5 à 10 chaînons dans lequel jusqu'à trois atomes du cycle peuvent être des hétéroatomes choisis indépendamment parmi azote, oxygène et soufre, le système de cycle étant éventuellement substitué par un ou plusieurs substituants choisis indépendamment parmi alkyle en C₁₋₄, alcoxy en C₁₋₄, halogène, trifluorométhyle, oxo, nitro, cyano, NR⁶R⁷ et -CH₂NR⁸R⁹ ;
R¹ et R² représentent chacun indépendamment un atome d'hydrogène, alkyle en C₁₋₆, alcényle en C₃₋₆, CH₂-cycloalkyle en C₃₋₅ ou cycloalkyle en C₃₋₆ ;
R³ représente un groupement X-Ar² ;
X représente un groupement S(O)ₙ, C(O) ou CH(OH) ;
n est 0, 1 ou 2 ;
Ar² représente un cycle pyrrolyle, thiazolyle, thiadiazolyle, oxazolyle, imidazolyle ou triazolyle, le cycle étant éventuellement substitué par un ou plusieurs substituants choisis indépendamment parmi alkyle en C₁₋₄, alcoxy en C₁₋₄, alkylthio en C₁₋₄, halogène, trifluorométhyle, oxo, hydroxy, amino, nitro, cyano et benzyle ;
R⁴ représente un atome d'hydrogène ou alkyle en C₁₋₄ ;
R⁵ représente un atome d'hydrogène ou un groupement hydroxy ;
R⁶ et R⁷ représentent chacun indépendamment un atome d'hydrogène ou alkyle en C₁₋₄, ou pris ensemble avec l'atome d'hydrogène auquel ils sont liés forment un cycle hétérocyclique saturé à 5 à 7 chaînons ;
R⁸ et R⁹ représentent chacun indépendamment un atome d'hydrogène ou alkyle en C₁₋₄, ou pris ensemble avec l'atome d'azote auquel ils sont liés forment un cycle hétérocyclique saturé à 5 à 7 chaînons ; et
Ar³ représente acénaphtényle, indanyle ou fluorényle, dont chacun peut être éventuellement substitué par un ou plusieurs substituants choisis indépendamment parmi alkyle en C₁₋₄, alcoxy en C₁₋₄, halogène ou trifluorométhyle ;
ou un sel ou un solvate pharmaceutiquement acceptable de celui-ci.

2. Composé selon la revendication 1, **caractérisé en ce que** R représente -C(R⁴) (R⁵)Ar¹.

3. Composé selon la revendication 1 ou la revendication 2, **caractérisé en ce que** Ar¹ représente phényle, naphtyle, pyrazolyle, thiényle, oxazolyle, imidazolyle, pyridinyle, pyridopyrrolyle, benzimidazolyle, indazolyle, benzothiazolyle, benzoxazolyle, thiazolyle ou benzotriazolyle, dont chacun peut être éventuellement substitué par un à quatre substituants choisis indépendamment parmi alkyle en C₁₋₄, alcoxy en C₁₋₄, halogène, trifluorométhyle, oxo, nitro, cyano, NR⁶R⁷ et -CH₂NR⁸R⁹.

4. Composé selon l'une quelconque des revendications 1 à 3, **caractérisé en ce que** R¹ et R² représentent chacun indépendamment un groupement alkyle en C₁₋₄.

5. Composé selon l'une quelconque des revendications 1 à 4, **caractérisé en ce que** Ar² représente thiazolyle ou triazolyle, dont chacun peut être éventuellement substitué par un ou trois substituants choisis indépendamment parmi alkyle en C₁₋₄.

6. Composé de formule (I), ou un sel ou un solvate pharmaceutiquement acceptable de celui-ci, selon la revendication 1, **caractérisé en ce qu'**il s'agit de :
1-Isobutyl-3-méthyl-5-[(1-méthyl-1H-pyrrol-2-yl)-carbonyl]-6-[2-(trifluorométhyl)benzyl]thiéno[2,3-d]pyrimidine-2,4(1H,3H)-dione,
3-Méthyl-1-(2-méthylpropyl)-5-(1H-1,2,4-triazol-3-ylthio)-6-[2-(trifluorométhyl)benzoyl]thiéno[2,3-d]pyrimidine-2,4(1H,3H)-dione,
3-Méthyl-1-(2-méthylpropyl)-5-(1H-1,2,4-triazol-3-ylthio)-6-[[2-(trifluorométhyl)phényl]méthyl]-thiéno[2,3-d]pyrimidine-2,4(1H,3H)-dione,
3-Méthyl-1-(2-méthylpropyl)-5-(1H-1,2,4-triazol-3-ylsulfonyl)-6-[[2-(trifluorométhyl)phényl]méthyl]-thiéno[2,3-d]pyrimidine-2,4(1H,3H)-dione,
3-Méthyl-1-(2-méthylpropyl)-5-(2-thiazolylthio)-6-[2-(trifluorométhyl)benzoyl]thiéno[2,3-d]-pyrimidine-2,4(1H,3H)-dione,
3-Méthyl-1-(2-méthylpropyl)-5-[[5-(méthylthio)-1,3,4-thiadiazol-2-yl]thio]-6-[2-(trifluorométhyl)benzoyl]thiéno[2,3-d]pyrimidine-2,4(1H,3H)-dione,
3-Méthyl-5-[(4-méthyl-2-oxazolyl)thio]-1-(2-méthylpropyl)-6-[2-(trifluorométhyl)benzoyl]-thiéno[2,3-d]pyrimidine-2,4(1H,3H)-dione,
3-Méthyl-1-(2-méthylpropyl)-5-[(5-méthyl-1H-1,2,4-triazol-3-yl)thio]-6-[2-(trifluorométhyl)-benzoyl]thiéno[2,3-d]pyrimidine-2,4(1H,3H)-dione,
3-Méthyl-1-(2-méthylpropyl)-5-[(5-hydroxy-4-(1-méthyléthyl)-1H-1,2,4-triazol-3-yl)thio]-6-[2-(trifluorométhyl)benzoyl]thiéno[2,3-d]pyrimidine-2,4(1H,3H)-dione,
3-Méthyl-1-(2-méthylpropyl)-5-[(5-méthyl-1,3,4-thiadiazol-2-yl)thio]-6-[2-(trifluorométhyl)-benzoyl]thiéno[2,3-d]pyrimidine-2,4(1H,3H)-dione,
5-(1H-Imidazol-2-ylthio)-3-méthyl-1-(2-méthyl-propyl)-6-[2-(trifluorométhyl)benzoyl]thiéno[2,3-d]pyrimidine-2,4(1H,3H)-dione,
5-[(4-(1,1-Diméthyléthyl)-4H-1,2,4-triazol-3-yl]thio]-3-méthyl-1-(2-méthylpropyl)-6-[2-(trifluorométhyl)benzoyl]thiéno[2,3-d]pyrimidine-2,4(1H,3H)-dione,
5-[(5-Amino-1,3,4-thiadiazol-2-yl)thio]-3-méthyl-1-(2-méthylpropyl)-6-[2-(trifluorométhyl)benzoyl]-thiéno[2,3-d]pyrimidine-2,4(1H,3H)-dione,
3-Méthyl-1-(2-méthylpropyl)-5-(1,3,4-thiadiazol-2-ylthio)-6-[2-(trifluorométhyl)benzoyl]thiéno[2,3-d]pyrimidine-2,4(1H,3H)-dione,
6-[Hydroxy-[2-(trifluorométhyl)phényl]méthyl]-5-(1H-imidazol-2-ylthio)-3-méthyl-1-(2-méthyl-propyl)thiéno[2,3-d]pyrimidine-2,4(1H,3H)-dione,
6-[Hydroxy-[2-(trifluorométhyl)phényl]méthyl]-3-méthyl-1-(2-méthylpropyl)-5-(1H-1,2,4-triazol-3-ylthio)thiéno[2,3-d]pyrimidine-2,4(1H,3H)-dione,
3-Méthyl-1-(2-méthylpropyl)-5-(2-thiazolylthio)-6-[[2-(trifluorométhyl)phényl]méthyl]thiéno[2,3-d]-pyrimidine-2,4(1H,3H)-dione,
Chlorhydrate de 3-méthyl-1-(2-méthylpropyl)-5-[(5-méthyl-1H-1,2,4-triazol-3-yl)thio]-6-[[2-(trifluorométhyl)phényl]méthyl]thiéno[2,3-d]-pyrimidine-2,4(1H,3H)-dione,
5-(1H-Imidazol-2-ylthio)-3-méthyl-1-(2-méthyl-propyl)-6-[[2-(trifluorométhyl)phényl]méthyl]-thiéno[2,3-d]pyrimidine-2,4(1H,3H)-dione,
3-Méthyl-1-(2-méthylpropyl)-5-(1,3,4-thiadiazol-2-ylthio)-6-[2-(trifluorométhyl)benzyl]thiéno[2,3-d]pyrimidine-2,4(1H,3H)-dione,
5-[(5-Amino-1,3,4-thiadiazol-2-yl)thio]-3-méthyl-1-(2-méthylpropyl)-6-[2-(trifluorométhyl)benzyl]-thiéno[2,3-d]pyrimidine-2,4(1H,3H)-dione,
3-Méthyl-1-(2-méthylpropyl)-5-[(5-méthyl-1H-1,2,4-triazol-3-yl)sulfonyl]-6-[[2-(trifluorométhyl)-phényl]méthyl]thiéno[2,3-d]pyrimidine-2,4(1H,3H)-dione,
5-(1H-Imidazol-2-ylsulfonyl)-3-méthyl-1-(2-méthyl-propyl)-6-[[2-(trifluorométhyl)phényl]méthyl]-thiéno[2,3-d]pyrimidine-2,4(1H,3H)-dione,
6-[Hydroxy-[2-(trifluorométhyl)phényl]méthyl]-3-méthyl-1-(2-méthylpropyl)-5-(1H-1,2,4-triazol-3-ylsulfonyl)thiéno[2,3-d]pyrimidine-2,4(1H,3H)-dione,
3-Méthyl-1-(2-méthylpropyl)-5-(1,3-thiazol-2-yl-sulfonyl)-6-[2-(trifluorométhyl)benzyl]thiéno[2,3-d]pyrimidine-2,4(1H,3H)-dione,
3-Méthyl-1-(2-méthylpropyl)-5-(1,3,4-thiadiazol-2-ylsulfonyl)-6-[2-(trifluorométhyl)benzyl]thiéno-[2,3-d]pyrimidine-2,4(1H,3H)-dione,
5-[(3-Bromo-1H-1,2,4-triazol-5-yl)thio]-3-méthyl-1-(2-méthylpropyl)-6-[2-(trifluorométhyl)phényl-méthyl]thiéno[2,3-d]pyrimidine-2,4(1H,3H)-dione,
3-Méthyl-1-(2-méthylpropyl)-5-[(5-oxo-4,5-dihydro-1H-1,2,4-triazol-3-yl)thio]-6-[2-(trifluoro-méthyl)phénylméthyl]thiéno[2,3-d]pyrimidine-2,4(1H,3H)-dione,
3-Méthyl-1-(1-méthyléthyl)-5-(1,3-thiazol-2-yl-thio)-6-[2-(trifluorométhyl)benzyl]thiéno[2,3-d]-pyrimidine-2,4(1H,3H)-dione,
3-Méthyl-1-(1-méthyléthyl)-5-(1,3-thiazol-2-yl-sulfonyl)-6-[2-(trifluorométhyl)benzyl]thiéno[2,3-d]pyrimidine-2,4(1H,3H)-dione,
6-(9H-Fluorén-9-yl)-3-méthyl-1-(2-méthylpropyl)-5-(1H-1,2,4-triazol-5-ylsulfonyl)thiéno[2,3-d]-pyrimidine-2,4(1H,3H)-dione,
6-(1H-Indol-3-ylcarbonyl)-3-méthyl-1-(2-méthyl-propyl)-5-(1H-1,2,4-triazol-3-ylthio)thiéno[2,3-d]pyrimidine-2,4(1H,3H)-dione,
6-(Hydroxy-(1H-indol-3-yl)méthyl)-3-méthyl-1-(2-méthylpropyl)-5-(1H-1,2,4-triazol-3-ylthio)thiéno-[2,3-d]pyrimidine-2,4(1H,3H)-dione,
3-Méthyl-1-(2-méthylpropyl)-6-(4-quinoléinyl-carbonyl)-5-(1,3-thiazol-2-ylthio)thiéno[2,3-d]-pyrimidine-2,4(1H,3H)-dione,
6-[Hydroxy-(4-quinoléinyl)méthyl]-3-méthyl-1-(2-méthylpropyl)-5-(1,3-thiazol-2-ylthio)thiéno[2,3-d]pyrimidine-2,4(1H,3H)-dione,
6-(1-Benzothién-3-ylméthyl)-3-méthyl-1-(2-méthyl-propyl)-5-(4H-1,2,4-triazol-3-ylthio)thiéno[2,3-d]pyrimidine-2,4(1H,3H)-dione,
ou un sel ou un solvate pharmaceutiquement acceptable de celui-ci.

7. Procédé de préparation d'un composé de formule (I) telle que définie dans la revendication 1, **caractérisé en ce qu'**il comprend,
(a) lorsque R représente C(O)Ar¹ et X représente un atome de soufre, la réaction d'un composé de formule générale 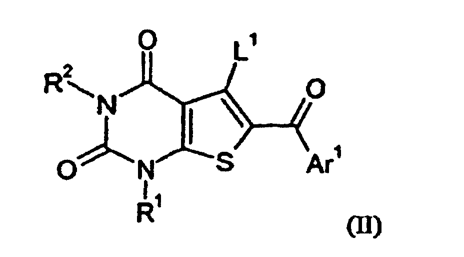 dans laquelle L¹ représente un groupement partant et R¹, R² et Ar¹ sont tels que définis dans la formule (I), avec un composé de formule générale (III), HS-Ar², dans laquelle Ar² est tel que défini dans la formule (I),en présence d'une base ; ou
(b) lorsque R représente -C(R⁴)(R⁵)Ar¹, R⁴ représente un atome d'hydrogène, R⁵ représente hydroxy et X représente un atome de soufre, la réaction d'un composé correspondant de formule (I) dans laquelle R représente C(O)Ar¹ avec un agent réducteur convenable ; ou
(c) lorsque R représente -C(R⁴)(R⁵)Ar¹, R⁴ représente un atome d'hydrogène, R⁵ représente un atome d'hydrogène et X représente un atome de soufre, la réaction d'un composé correspondant de formule (I) dans laquelle R représente C(O)Ar¹ avec un agent réducteur convenable, en présence d'un acide de Lewis ; ou
(d) lorsque R représente -C(R⁴) (R⁵)Ar¹ et R⁴ représente alkyle en C₁₋₄, la réaction d'un composé correspondant de formule (I) dans laquelle R représente C(O)Ar¹ avec un réactif de Grignard convenable, suivie d'une réaction avec un agent déshydratant en présence d'une base, puis suivie d'une réaction d'hydrogénation ; ou
(e) lorsque X représente SO ou SO₂, la réaction d'un composé correspondant de formule (I) dans laquelle X est un atome de soufre avec un agent oxydant convenable ; ou
(f) lorsque X représente CH(OH), la réaction d'un composé de formule générale 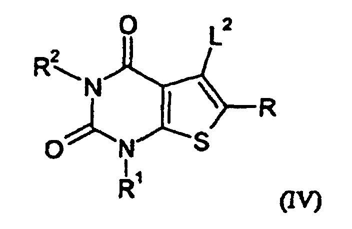 dans laquelle L² représente un groupement partant et R, R¹ et R² sont tels que définis dans la formule (I), avec un réactif de Grignard convenable et ensuite avec un composé de formule générale (V), Ar²CHO, dans laquelle Ar² est tel que défini dans la formule (I) ; ou
(g) lorsque X représente C(O), la réaction d'un composé correspondant de formule (I) dans laquelle X représente CH(OH) avec un agent oxydant convenable ; ou
(h) lorsque X représente un atome de soufre, la réaction d'un composé de formule (IV) tel que définie dans (f) ci-dessus avec un composé d'alkyllithium convenable ou un réactif de Grignard convenable et ensuite avec un composé de formule générale (VI), Ar²-S-S-Ar², dans laquelle Ar² est tel que défini dans la formule (I) ;
et éventuellement après (a), (b), (c), (d), (e), (f), (g) ou (h) la formation d'un sel ou d'un solvate pharmaceutiquement acceptable du composé de formule (I) obtenu.

8. Composition pharmaceutique comprenant un composé de formule (I) ou un sel ou un solvate pharmaceutiquement acceptable de celui-ci selon l'une quelconque des revendications 1 à 6 en association avec un adjuvant, un diluant ou un support pharmaceutiquement acceptable.

9. Procédé de préparation d'une composition pharmaceutique selon la revendication 8, **caractérisé en ce qu'**il comprend le mélange d'un composé de formule (I) ou un sel ou un solvate pharmaceutiquement acceptable de celui-ci selon l'une quelconque des revendications 1 à 6 avec un adjuvant, un diluant ou un support pharmaceutiquement acceptable.

10. Composé de formule (I) ou un sel ou un solvate pharmaceutiquement acceptable de celui-ci selon l'une quelconque des revendications 1 à 6 destiné à être utilisé en thérapie.

11. Utilisation d'un composé de formule (I) ou un sel ou un solvate pharmaceutiquement acceptable de celui-ci selon l'une quelconque des revendications 1 à 6 dans la fabrication d'un médicament destiné à être utilisé en thérapie.

12. Utilisation d'un composé de formule (I) ou un sel ou un solvate pharmaceutiquement acceptable de celui-ci selon l'une quelconque des revendications 1 à 6 dans la fabrication d'un médicament destiné à être utilisé dans le traitement d'une maladie obstructive des voies aériennes.

13. Utilisation selon la revendication 12, **caractérisée en ce que** la maladie obstructive des voies aériennes est l'asthme ou la bronchopneumopathie chronique obstructive.

14. Utilisation d'un composé de formule (I) ou un sel ou un solvate pharmaceutiquement acceptable de celui-ci selon l'une quelconque des revendications 1 à 6 dans la fabrication d'un médicament destiné à être utilisé dans le traitement du rejet d'allogreffe.
